# EUROPÄISCHE PATENTSCHRIFT

(11) **EP 2 327 417 B1**
(45) Veröffentlichungstag und Bekanntmachung des Hinweises auf die Patenterteilung: **06.05.2015**
(21) Anmeldenummer: 10010912.3
(22) Anmeldetag: 23.03.2006
(51) Int. Cl.: A61K 39/00, C07K 14/47, A61K 48/00

(54) **Identifizierung von Oberflächen-assoziierten Antigenen für die Tumordiagnose und- therapie**
Identification of surface-associated antigens for tumour diagnosis and therapy
Identification d'antigènes de surfaces pour le diagnostic et la thérapie de tumeurs

(30) Priorität: 24.03.2005 DE 102005013846
(43) Veröffentlichungstag der Anmeldung: 01.06.2011
(62) Teilanmeldung aus: 06723678.6
(73) Patentinhaber: BioNTech AG, 55131 Mainz (DE)
(72) Erfinder: Sahin, Ugur, 55116 Mainz (DE); Türeci, Özlem, 55116 Mainz (DE); Koslowski, Michael, 65933 Frankfurt (DE); Helftenbein, Gerd, 35329 Gemünden (DE); Usener, Dirk, 65193 Wiesbaden (DE); Schlüter, Volker, 82061 Neuried (DE)
(74) Vertreter: Schnappauf, Georg

(56) Entgegenhaltungen:
- WO-A-03/076631
- WO-A2-02/12343
- WO-A2-2004/047863
- WO-A2-2005/030250
- US-A1- 2002 198 371
- L. BUONAGURO ET AL: 'Translating Tumor Antigens into Cancer Vaccines' CLINICAL AND VACCINE IMMUNOLOGY Bd. 18, Nr. 1, 01 Januar 2011, Seiten 23 - 34, XP055009295 DOI: 10.1128/CVI.00286-10 ISSN: 1556-6811
- DATABASE EMBL [Online] 25 März 2004 'Homo sapiens cDNA clone IMAGE:30347757.' Retrieved from EBI, accession no. EM_HUM:BC067882 Database accession no. BC067882

### SEQUENCE LISTING

<110> BioNTech AG
<120> Identifizierung von Oberflächen-assoziierten Antigenen für die Tumordiagnose und -therapie
<130> 342-26 EPT1
<140> EP 10 010 912.3
<150> DE 10 2005 013 846.2
   <151> 2005-03-24
<160> 8
<170> PatentIn version 3.3
<210> 5
   <211> 659
   <212> DNA
   <213> Homo sapiens
<400> 5
   ggaagcagag ctgaggaagg cagccactgc ccacccatgg ctaagcaggg agaaatgaat      60
   acctcaacct cactttcccc ctaccccaag tctcccagca agtctcctac cagaaggcag     120
   agggcagggc agctctttga cacaatccac aaagatcagc ccttctggag ctcagagcta     180
   ttacagagaa gcctggagag tgtgtcaaga cagcttttct ccaggtggtg tgacgctgct     240
   gctaataatg gtgatgacat caaaactgcc aagggcctta gaaatctgct ggtccagcac     300
   gaagcaggga gatcaccaaa aaggggaggc agagctggaa gagaacacag ctgtcctgcc     360
   atccaggtgc tccattatgc ctgaaaggtt aacttcacca ccaaagccca agaagaggtt     420
   ttcttcgcca aagatgggga agtgctgaca acgtttgaca ttaaaaacat ctatgttctc     480
   ccagacctgt caggacagac agccattgtt ggacactttg acttcagagc accttctgga     540
   aaagagcttc tgttggatga cagcgcaatt gtctgggcag aaggaccctt aaagattaga     600
   gctgagagaa ccctaagaac caagaccaca cagcacctct cacatcccaa gctccagga      659
<210> 6
   <211> 115
   <212> PRT
   <213> Homo sapiens
<400> 6
   Met Ala Lys Gln Gly Glu Met Asn Thr Ser Thr Ser Leu Ser Pro Tyr
   1               5                   10                  15
   Pro Lys Ser Pro Ser Lys Ser Pro Thr Arg Arg Gln Arg Ala Gly Gln
                   20                  25                  30
   Leu Phe Asp Thr Ile His Lys Asp Gln Pro Phe Trp Ser Ser Glu Leu
           35                  40                  45
   Leu Gln Arg Ser Leu Glu Ser Val Ser Arg Gln Leu Phe Ser Arg Trp
       50                  55                  60
   Cys Asp Ala Ala Ala Asn Asn Gly Asp Asp Ile Lys Thr Ala Lys Gly
   65                  70                  75                  80
   Leu Arg Asn Leu Leu Val Gln His Glu Ala Gly Arg Ser Pro Lys Arg
                   85                  90                  95
   Gly Gly Arg Ala Gly Arg Glu His Ser Cys Pro Ala Ile Gln Val Leu
               100                 105                 110
   His Tyr Ala
           115
<210> 7
   <211> 22
   <212> DNA
   <213> künstliche Sequenz
<220>
   <223> Oligonukleotid
<400> 7
   aggtggtgtg acgctgctgc ta 22
<210> 8
   <211> 22
   <212> DNA
   <213> künstliche Sequenz
<220>
   <223> Oligonukleotid
<400> 8
   tcttcttggg ctttggtggt ga 22
<210> 269
<210> 269
   <211> 2856
   <212> DNA
   <213> Homo sapiens
<400> 269
   atggccaaaa gaaatctcag cactgtgaca gagttcattc ttgtagtctt cacagatcac 60
   cctgaactgg cagttccact cttcctagtg tttctcagtt tctatcttgt cacttttctg 120
   gggaatgggg ggatgatcat tctaatccaa gtggatgccc aactccacac ccccgtgtac 180
   ttcttcctga gccaccttgc tttcctggat gcctgctgtg cctcagtaat cacccctcag 240
   attctggcca cactggccac agacaagaca gttatctcct atggctgccg tgctgtgcag 300
   ttctctttct tcaccatatg tgcaggcaca gagtgttacc tgctgtcagt gatggcctat 360
   gaccgctttg ttgccattag caatccactg cactgtaaca tgaccatgac tccaggtacc 420
   tgcagggtct ttttggccag tgccttcatc tgtggggtgt caggggccat tctgcatacc 480
   acgtgcacct tcaccctctc cttctgttgt gacaatcaga tcaacttctt cttctgtgac 540
   ctcccacccc tgctgaagct cgcctgcagc agcatgacac aaactgagat tgtcattctc 600
   ctttgtgcaa aatgcatgtt cctagccaat gtcatggtta tcctgatctg ctacatgctc 660
   attatcagag ccattttgag ggtgaagtcg gcaggaggcc tcctgatagc atctgctcat 720
   ttcgatgcat atgtatatga gacaggcatc aactacaaca cagtttatgg ctcaggaaag 780
   gcagtagggt ggtcctggag gagcctgcgg gaaaccaacc acatgagacc aggaaatact 840
   tcaaaacact cagcagccca gctgcatcaa tgcctcatcc agcaagttgg caggtggccc 900
   ttgcagagca tgcccttccc cgtttctgca gggccacctt ataagtcagt gcagcctctc 960
   cctggagacc cccggcctct cctgtgcatc accggattat ttctgacttt gaagatgatg 1020
   gggtgtgggc ccaggaggcc cagggacagg aagtctgact tcttcataaa cacagaccct 1080
   ggtgcagggt caccagaaga acagaggtgt ggatgggaag ggcatccttc ccactcctat 1140
   accctggggc tgtctctgcc agtcaacttc ggcctgaaat gtccatggtg gacactatct 1200
   ggacccccag ctacctgcca acgtccagac ctgcagacac cttctccacc aaaggagata 1260
   tgttcatccg ggctgcgacc ccttacacac agcgctggac cagacagaag tcaagttcca 1320
   gcagcctccg gagcagccac tatgctgaca aaggggctgc ccgacatcac tgtgggactg 1380
   cagatttatg actcctgcat ctcagggatc caggctctgg ggagcaccct ggccctgctg 1440
   tccaatcagc ttccacccac aaccaactat gcttgtggct cccagcaaca tctcctgggc 1500
   gtggttggag ggatgacctt cctggagtca gagcccatgt ctgagctgct ctccatctac 1560
   agagtccctc agggccaaag actcaccaaa aactttgaag taaaagaact tgtctgcaca 1620
   tatctggtag gacagcttcc ttatggcctg gtcagttatg acaacagcaa ctttgagtgg 1680
   ctggatcagc agctgcagaa gcagatcggg ggcgagggac ttcctgttgg cgctgcgccc 1740
   agccgtgtag ccaggcaaca gtctgatgag gaagctgtgg gaggagtgca gggatacagg 1800
   tggtctggat taggggcttc catccaaagt gccagagaag gggcttggca tcgcacaggg 1860
   ctggagaaca tgaccactgc ccacctgtct gccttcaaac ttcctgatct aactgccact 1920
   taccaagcct acctggcagc caaagccctg tgggttgcct atcagaactt gatgtcctgc 1980
   tctgagagag agggaccatt cctgggaggc acgtatgcca atgcatggga agccaggctt 2040
   tctcaggtta acttcaccac caaagcccaa gaagaggttt tcttcgccaa agatggggaa 2100
   gtgctgacaa cgtttgacat taaaaacatc tatgttctcc cagacctgtc aggacagaca 2160
   gccattgttg gacactttga cttcagagca ccttctggaa aagagcttct gttggatgac 2220
   agcgcaattg tctgggcaga aggaccctta aagattagag ctgagagaac cctaagaacc 2280
   aagaccacac agcacctctc acatcccaag ctccaggagt cccttcctct gtctgcaacg 2340
   aaaaacgtcc tgtggaaacc aggaagtcaa ccctatttga gaagtcaaaa tgctgctaca 2400
   aaagccttcc ctgacccaga agagaaatcg caatgtcacc agtttctctt tctcccttca 2460
   gatagtgttg catgtcagaa gtgctctgac aaccagtggc ccaatgtgca gaagggcgag 2520
   tgcatcccca aaacccttga cttcttgttc tatcacaagc cccttgacac agcgttggct 2580
   gtctgcacag ccctgctctt tctccttgcc ctggccatct taggcatctt ccatgttgtc 2640
   tgctcctgtg tctgggtgtc cttcatacct gcccacatgc atgcccacag caaagacacc 2700
   atggccatgg aggtctttgt catcttggca tcagcaggag gcctcatgtc ctccctcttc 2760
   ttttccaaat gctacatcat ccttctccat cctgaaaaga acacaaaaga ccaaatgttt 2820
   ggccggcatc atcgcaagtg ggaaaaactg aagtga 2856
<210> 270
   <211> 951
   <212> PRT
   <213> Homo sapiens
<400> 270
   Met Ala Lys Arg Asn Leu Ser Thr Val Thr Glu Phe Ile Leu Val Val
   1               5                   10                  15
   Phe Thr Asp His Pro Glu Leu Ala Val Pro Leu Phe Leu Val Phe Leu
               20                  25                  30
   Ser Phe Tyr Leu Val Thr Phe Leu Gly Asn Gly Gly Met Ile Ile Leu
           35                  40                  45
   Ile Gln Val Asp Ala Gln Leu His Thr Pro Val Tyr Phe Phe Leu Ser
       50                  55                  60
   His Leu Ala Phe Leu Asp Ala Cys Cys Ala Ser Val Ile Thr Pro Gln
   65                  70                  75                  80
   Ile Leu Ala Thr Leu Ala Thr Asp Lys Thr Val Ile Ser Tyr Gly Cys
                   85                  90                  95
   Arg Ala Val Gln Phe Ser Phe Phe Thr Ile Cys Ala Gly Thr Glu Cys
               100                 105                 110
   Tyr Leu Leu Ser Val Met Ala Tyr Asp Arg Phe Val Ala Ile Ser Asn
           115                 120                 125
   Pro Leu His Cys Asn Met Thr Met Thr Pro Gly Thr Cys Arg Val Phe
       130                 135                 140
   Leu Ala Ser Ala Phe Ile Cys Gly Val Ser Gly Ala Ile Leu His Thr
   145                 150                 155                 160
   Thr Cys Thr Phe Thr Leu Ser Phe Cys Cys Asp Asn Gln Ile Asn Phe
                   165                 170                 175
   Phe Phe Cys Asp Leu Pro Pro Leu Leu Lys Leu Ala Cys Ser Ser Met
               180                 185                 190
   Thr Gln Thr Glu Ile Val Ile Leu Leu Cys Ala Lys Cys Met Phe Leu
           195                 200                 205
   Ala Asn Val Met Val Ile Leu Ile Cys Tyr Met Leu Ile Ile Arg Ala
       210                 215                 220
   Ile Leu Arg Val Lys Ser Ala Gly Gly Leu Leu Ile Ala Ser Ala His
   225                 230                 235                 240
   Phe Asp Ala Tyr Val Tyr Glu Thr Gly Ile Asn Tyr Asn Thr Val Tyr
                   245                 250                 255
   Gly Ser Gly Lys Ala Val Gly Trp Ser Trp Arg Ser Leu Arg Glu Thr
               260                 265                 270
   Asn His Met Arg Pro Gly Asn Thr Ser Lys His Ser Ala Ala Gln Leu
           275                 280                 285
   His Gln Cys Leu Ile Gln Gln Val Gly Arg Trp Pro Leu Gln Ser Met
       290                 295                 300
   Pro Phe Pro Val Ser Ala Gly Pro Pro Tyr Lys Ser Val Gln Pro Leu
   305                 310                 315                 320
   Pro Gly Asp Pro Arg Pro Leu Leu Cys Ile Thr Gly Leu Phe Leu Thr
                   325                 330                 335
   Leu Lys Met Met Gly Cys Gly Pro Arg Arg Pro Arg Asp Arg Lys Ser
               340                 345                 350
   Asp Phe Phe Ile Asn Thr Asp Pro Gly Ala Gly Ser Pro Glu Glu Gln
           355                 360                 365
   Arg Cys Gly Trp Glu Gly His Pro Ser His Ser Tyr Thr Leu Gly Leu
       370                 375                 380
   Ser Leu Pro Val Asn Phe Gly Leu Lys Cys Pro Trp Trp Thr Leu Ser
   385                 390                 395                 400
   Gly Pro Pro Ala Thr Cys Gln Arg Pro Asp Leu Gln Thr Pro Ser Pro
                   405                 410                 415
   Pro Lys Glu Ile Cys Ser Ser Gly Leu Arg Pro Leu Thr His Ser Ala
               420                 425                 430
   Gly Pro Asp Arg Ser Gln Val Pro Ala Ala Ser Gly Ala Ala Thr Met
           435                 440                 445
   Leu Thr Lys Gly Leu Pro Asp Ile Thr Val Gly Leu Gln Ile Tyr Asp
       450                 455                 460
   Ser Cys Ile Ser Gly Ile Gln Ala Leu Gly Ser Thr Leu Ala Leu Leu
   465                 470                 475                 480
   Ser Asn Gln Leu Pro Pro Thr Thr Asn Tyr Ala Cys Gly Ser Gln Gln
                   485                 490                 495
   His Leu Leu Gly Val Val Gly Gly Met Thr Phe Leu Glu Ser Glu Pro
               500                 505                 510
   Met Ser Glu Leu Leu Ser Ile Tyr Arg Val Pro Gln Gly Gln Arg Leu
           515                 520                 525
   Thr Lys Asn Phe Glu Val Lys Glu Leu Val Cys Thr Tyr Leu Val Gly
       530                 535                 540
   Gln Leu Pro Tyr Gly Leu Val Ser Tyr Asp Asn Ser Asn Phe Glu Trp
   545                 550                 555                 560
   Leu Asp Gln Gln Leu Gln Lys Gln Ile Gly Gly Glu Gly Leu Pro Val
                   565                 570                 575
   Gly Ala Ala Pro Ser Arg Val Ala Arg Gln Gln Ser Asp Glu Glu Ala
               580                 585                 590
   Val Gly Gly Val Gln Gly Tyr Arg Trp Ser Gly Leu Gly Ala Ser Ile
           595                 600                 605
   Gln Ser Ala Arg Glu Gly Ala Trp His Arg Thr Gly Leu Glu Asn Met
       610                 615                 620
   Thr Thr Ala His Leu Ser Ala Phe Lys Leu Pro Asp Leu Thr Ala Thr
   625                 630                 635                 640
   Tyr Gln Ala Tyr Leu Ala Ala Lys Ala Leu Trp Val Ala Tyr Gln Asn
                   645                 650                 655
   Leu Met Ser Cys Ser Glu Arg Glu Gly Pro Phe Leu Gly Gly Thr Tyr
               660                 665                 670
   Ala Asn Ala Trp Glu Ala Arg Leu Ser Gln Val Asn Phe Thr Thr Lys
           675                 680                 685
   Ala Gln Glu Glu Val Phe Phe Ala Lys Asp Gly Glu Val Leu Thr Thr
       690                 695                 700
   Phe Asp Ile Lys Asn Ile Tyr Val Leu Pro Asp Leu Ser Gly Gln Thr
   705                 710                 715                 720
   Ala Ile Val Gly His Phe Asp Phe Arg Ala Pro Ser Gly Lys Glu Leu
                   725                 730                 735
   Leu Leu Asp Asp Ser Ala Ile Val Trp Ala Glu Gly Pro Leu Lys Ile
               740                 745                 750
   Arg Ala Glu Arg Thr Leu Arg Thr Lys Thr Thr Gln His Leu Ser His
           755                 760                 765
   Pro Lys Leu Gln Glu Ser Leu Pro Leu Ser Ala Thr Lys Asn Val Leu
   770 775 780
   Trp Lys Pro Gly Ser Gln Pro Tyr Leu Arg Ser Gln Asn Ala Ala Thr
   785 790 795 800
   Lys Ala Phe Pro Asp Pro Glu Glu Lys Ser Gln Cys His Gln Phe Leu
   805 810 815
   Phe Leu Pro Ser Asp Ser Val Ala Cys Gln Lys Cys Ser Asp Asn Gln
   820 825 830
   Trp Pro Asn Val Gln Lys Gly Glu Cys Ile Pro Lys Thr Leu Asp Phe
   835 840 845
   Leu Phe Tyr His Lys Pro Leu Asp Thr Ala Leu Ala Val Cys Thr Ala
   850 855 860
   Leu Leu Phe Leu Leu Ala Leu Ala Ile Leu Gly Ile Phe His Val Val
   865 870 875 880
   Cys Ser Cys Val Trp Val Ser Phe Ile Pro Ala His Met His Ala His
   885 890 895
   Ser Lys Asp Thr Met Ala Met Glu Val Phe Val Ile Leu Ala Ser Ala
   900 905 910
   Gly Gly Leu Met Ser Ser Leu Phe Phe Ser Lys Cys Tyr Ile Ile Leu
   915 920 925
   Leu His Pro Glu Lys Asn Thr Lys Asp Gln Met Phe Gly Arg His His
   930 935 940
   Arg Lys Trp Glu Lys Leu Lys
   945 950
<210> 282
   <211> 13
   <212> PRT
   <213> Homo sapiens
<400> 282
   Met Ala Lys Gln Gly Glu Met Asn Thr Ser Thr Ser Cys
   1 5 10
<210> 283
   <211> 13
   <212> PRT
   <213> Homo sapiens
<400> 283
   Pro Lys Arg Gly Gly Arg Ala Gly Arg Glu His Ser Cys
   1               5                   10

## Beschreibung

Trotz interdisziplinärer Ansätze und Ausreizung klassischer Therapiemodalitäten gehören Krebserkrankungen weiterhin zu den führenden Todesursachen. Neuere therapeutische Konzepte zielen darauf ab, das patienteneigene Immunsystem durch Einsatz von rekombinanten Tumorvakzinen und anderen spezifischen Maßnahmen wie Antikörpertherapie in das therapeutische Gesamtkonzept mit einzubeziehen. Voraussetzung für den Erfolg einer solchen Strategie ist die Erkennung von Tumor-spezifischen oder Tumor-assoziierten Antigenen bzw. Epitopen durch das Immunsystem des Patienten, dessen Effektorfunktionen interventionell verstärkt werden sollen. Tumorzellen unterscheiden sich biologisch wesentlich von ihren nichtmalignen Ursprungszellen. Diese Differenzen sind durch während der Tumorentwicklung erworbene genetische Veränderungen bedingt und führen u.a. auch zur der Bildung qualitativ oder quantitativ veränderter molekularer Strukturen in den Krebszellen. Werden solche Tumor-assoziierten Strukturen vom spezifischen Immunsystem des tumortragenden Wirtes erkannt, spricht man von Tumor-assoziierten Antigenen.

An der spezifischen Erkennung von Tumor-assoziierten Antigenen sind zelluläre und humorale Mechanismen beteiligt, die zwei miteinander funktionell vernetzte Einheiten darstellen: CD4⁺ und CD8⁺ T-Lymphozyten erkennen prozessierte Antigene, die auf den Molekülen der MHC- (Major Histocompatibility complex = Histokompatibilitäts-Antigene) Klassen II bzw. I präsentiert werden, während B-Lymphozyten zirkulierende Antikörpermoleküle produzieren, die direkt an unprozessierte Antigene binden.

Die potentielle klinisch-therapeutische Bedeutung von Tumor-assoziierten Antigenen ergibt sich aus der Tatsache, dass die Erkennung von Antigenen auf neoplastischen Zellen durch das Immunsystem zur Initiierung von cytotoxischen Effektormechanismen führt und bei Vorhandensein von T-Helferzellen die Elimination der Krebszellen bewirken kann (Pardoll, Nat. Med. 4:525-31, 1998). Entsprechend ist es eine zentrale Zielsetzung der Tumorimmunologie, diese Strukturen molekular zu definieren. Die molekulare Natur dieser Antigene blieb lange enigmatisch. Erst als entsprechende Klonierungstechniken entwickelt wurden, gelang es, durch Analyse der Zielstrukturen von cytotoxischen T-Lymphozyten (CTL) (van der Bruggen et al., Science 254:1643-7, 1991) bzw. mit zirkulierenden Autoantikörpern (Sahin et al., Curr. Opin. Immunol. 9:709-16, 1997) als Sonden cDNA-Expressionbanken von Tumoren systematisch auf Tumor-assoziierte Antigene zu screenen. Hierzu wurden cDNA-Expressionsbanken aus frischem Tumorgewebe hergestellt und in geeigneten Systemen als Proteine rekombinant exprimiert. Aus Patienten isolierte Immuneffektoren, nämlich CTL-Klone mit Tumor-spezifischem Lysemuster, oder zirkulierende Autoantikörper wurden genutzt, um die respektiven Antigene zu klonieren.

Durch diese Ansätze sind in den letzten Jahren eine Vielzahl von Antigenen in verschiedenen Neoplasien definiert worden. Von großem Interesse ist dabei die Klasse der Cancer/Testis-Antigene (CTA). CTA und sie kodierende Gene (Cancer/Testis-Gene oder CTG) sind durch ihr charakteristisches Expressionsmuster definiert (Türeci et al., Mol. Met. Today 3:342-9, 1997). Sie finden sich nicht in Normalgeweben bis auf Testis bzw. Keimzellen, werden jedoch in einer Reihe von humanen Malignomen exprimiert und zwar nicht tumortypspezifisch, sondern mit unterschiedlicher Häufigkeit in Tumorentitäten ganz unterschiedlicher Herkunft (Chen & Old, Cancer J. Sci. Am. 5:16-7, 1999). Auch Serumreaktivitäten gegen CTA finden sich nicht in gesunden Kontrollen, sondern lediglich in Tumorpatienten. Insbesondere aufgrund ihrer Gewebeverteilung ist diese Antigenklasse von besonderem Wert für immuntherapeutische Vorhaben und wird in derzeit laufenden klinischen Patientenstudien getestet (Marchand et al., Int. J. Cancer 80:219-30, 1999; Knuth et al., Cancer Chemother. Pharmacol. 46: S46-51, 2000).

Allerdings nutzen die oben dargestellten klassischen Verfahren zur Antigenidentifizierung Immuneffektoren (zirkulierende Autoantikörper oder CTL-Klone) aus Patienten mit in der Regel bereits fortgeschrittenem Krebs als Sonden. Aus einer Reihe von Daten geht hervor, dass Tumore z.B. zur Tolerisierung und Anergisierung von T-Zellen führen können und gerade im Verlauf der Erkrankung diejenigen Spezifitäten aus dem Immuneffektorenrepertoire verloren gehen, die eine effektive Immunerkennung bewirken könnten. Aus laufenden Patientenstudien hat sich noch kein gesicherter Beweis für eine tatsächliche Wirkung der bisher entdeckten und genutzten Tumor-assoziierten Antigene ergeben. Entsprechend kann nicht ausgeschlossen werden, dass spontane Immunantworten evozierende Proteine die falschen Zielstrukturen sind.

Es war die Aufgabe der vorliegenden Erfindung Zielstrukturen für eine Diagnose und Therapie von Krebserkrankungen bereitzustellen.

Diese Aufgabe wird erfindungsgemäß durch den Gegenstand der Patentansprüche gelöst.

Die vorliegende Erfindung basiert auf einer Strategie für eine Identifizierung und Bereitstellung Tumor-assoziiert exprimierter Antigene und der dafür kodierenden Nukleinsäuren. Diese Strategie beruht auf der Auswertung humaner Protein- und Nukleinsäuredatenbanken im Hinblick auf potenzielle, auf der Zelloberfläche zugängliche, krebsspezifische Antigene. Die Definition der dafür notwendigen Filterkriterien zusammen mit einer Hochdurchsatz-Methodik zur Analyse möglichst aller Proteine bilden den zentralen Bestandteil der Strategie. Durch Datamining wird zunächst eine möglichst komplette Liste aller bekannter Gene aufgestellt, die dem Grundprinzip Gen zu mRNA zu Protein folgend auf das Vorhandensein einer oder mehrerer Transmembrandomänen hin untersucht werden. Hieran schließen sich eine Homologiesuche, eine Einteilung der Treffer in gewebsspezifische Gruppen (u.a. Tumorgewebe) und eine Überprüfung der realen Existenz der mRNA an. Schließlich werden die so identifizierten Proteine z.B. durch Expressionsanalysen und proteinchemische Verfahren auf ihre aberrante Aktivierung in Tumoren evaluiert.

Datamining ist ein bekanntes Verfahren zur Identifizierung von Tumor-assoziierten Genen. Bei den herkömmlichen Strategien werden allerdings in der Regel Transkriptome von Normalgewebebanken elektronisch von Tumorgewebsbanken subtrahiert unter der Annahme, dass die verbleibenden Gene Tumor-spezifisch sind (Schmitt et al., Nucleic Acids Res. 27:4251-60, 1999; Vasmatzis et al., Proc. Natl. Acad. Sci. USA. 95:300-4, 1998; Scheurle et al., Cancer Res. 60:4037-43, 2000).

Das hierin beschriebene Konzept, das sich als viel erfolgreicher erwiesen hat, beruht jedoch darauf, Datamining zur elektronischen Extraktion aller Gene, die für auf der Zelloberfläche zugängliche, krebsspezifische Antigene kodieren, zu nutzen und diese sodann auf ektope Expression in Tumoren zu evaluieren.

Somit wird vorliegend eine Strategie zur Identifizierung von differentiell in Tumoren exprimierten Genen beschrieben. Diese kombiniert Datamining von öffentlichen Sequenzbanken ("*in silico*") mit darauffolgenden evaluierenden labor-experimentellen ("wet bench") Untersuchungen.

Eine kombinierte Strategie basierend auf unterschiedlichen bioinformatischen Skripten ermöglichte die Identifizierung von Genen, die für auf der Zelloberfläche zugängliche, krebsspezifische Antigene kodieren. Die Identifizierung und Bereitstellung dieser Tumor-assoziierten Gene und der dadurch kodierten Genprodukte erfolgte unabhängig von einer immunogenen Wirkung.

Die gemäß der hierin beschriebenen Strategie identifizierten Tumor-assoziierten Antigene weisen eine Aminosäuresequenz auf, die von einer Nukleinsäure kodiert wird, die aus der Gruppe ausgewählt ist bestehend aus (a) einer Nukleinsäure, die eine Nukleinsäuresequenz gemäß SEQ ID NO: 5 des Sequenzprotokolls umfasst und (b) einer Nukleinsäure, die zu der Nukleinsäure unter (a) mindestens 90% Sequenzidentität aufweist. In einer bevorzugten Ausführungsform weist ein gemäß der hierin beschriebenen Strategie identifiziertes Tumor-assoziiertes Antigen eine Aminosäuresequenz auf, die von einer Nukleinsäure gemäß SEQ ID NO: 5 des Sequenzprotokolls kodiert wird. In einer weiteren bevorzugten Ausführungsform umfasst ein gemäß der hierin beschriebenen Strategie identifiziertes Tumor-assoziiertes Antigen eine Aminosäuresequenz, die aus der Gruppe ausgewählt ist, bestehend aus SEQ ID NOs: 6, 282 und 283 des Sequenzprotokolls.

Die vorliegende Erfindung betrifft allgemein die Verwendung von gemäß der hierin beschriebenen Strategie identifizierten Tumor-assoziierten Antigenen oder von dafür kodierenden Nukleinsäuren für die Diagnose. Darüber hinaus wird zusätzlich deren Verwendung zur Therapie offenbart. Diese Nutzung kann einzelne, aber auch Kombinationen von mehreren dieser Antigene, funktionalen Fragmente, Nukleinsäuren, Antikörper etc. betreffen, in einer Ausführungsform auch in Kombination mit anderen Tumor-assoziierten Genen und Antigenen für eine Diagnose, Therapie und Verlaufskontrolle.

Die Eigenschaft der gemäß der hierin beschriebenen Strategie identifizierten Tumor-assoziierten Antigene, dass sie auf oder an der Zelloberfläche lokalisiert sind, qualifiziert sie als geeignete Ziele oder Mittel für die Therapie und Diagnose. Besonders geeignet hierfür ist ein Teil der gemäß der hierin beschriebenen Strategie identifizierten Tumor-assoziierten Antigene, der dem Nichttransmembrananteil, insbesondere dem extrazellulären Anteil der Antigene entspricht oder davon umfasst wird. Somit ist ein Teil der identifizierten Tumor-assoziierten Antigene, der dem Nichttransmembrananteil der Antigene entspricht oder davon umfasst ist, oder ein entsprechender Teil der für die gemäß der hierin beschriebenen Strategie identifizierten Antigene kodierenden Nukleinsäuren für eine Diagnose bevorzugt. Ähnlich ist die Verwendung von Antikörpern bevorzugt, die gegen einen Teil der gemäß der hierin beschriebenen Strategie identifizierten Tumor-assoziierten Antigene gerichtet sind, der dem Nichttransmembrananteil der Antigene entspricht oder davon umfasst ist.

Bevorzugte Erkrankungen für eine Diagnose sind solche, bei denen eine selektive Expression oder abnormale Expression von einem oder mehreren der gemäß der hierin beschriebenen Strategie identifizierten Tumor-assoziierten Antigene vorliegt.

Vorliegend werden auch Genprodukte beschrieben, d.h. Nukleinsäuren und Proteine bzw. Peptide, die Tumor-assoziiert exprimiert werden und durch verändertes Spleißen (Spleißvarianten) bekannter Gene bzw. durch veränderte Translation unter Nutzung alternativer offener Leserahmen entstehen. In diesem Aspekt werden Nukleinsäuren offenbart, die eine Nukleinsäuresequenz ausgewählt aus der Gruppe bestehend aus den Sequenzen gemäß SEQ ID NOs: 5 und 269 des Sequenzprotokolls umfassen. Ferner bezieht sich dieser Aspekt auf Proteine bzw. Peptide, die eine Aminosäuresequenz, ausgewählt aus der Gruppe, bestehend aus den Sequenzen gemäß SEQ ID NOs: 6, 270, 282 und 283 des Sequenzprotokolls, umfassen. Diese Spleißvarianten sind als Targets für die Diagnostik und Therapie von Tumorerkrankungen verwendbar.

Für die Entstehung von Spleißvarianten können verschiedenste Mechanismen ursächlich sein, beispielsweise
- die Nutzung variabler Transkriptionsinitiationsstellen
- die Nutzung zusätzlicher Exons
- vollständiges oder unvollständiges Ausspleißen von einzelnen oder mehreren Exons,
- über Mutation veränderte Spleißregulatorsequenzen (Deletion bzw. Schaffung neuer Donor/Akzeptorsequenzen),
- die unvollständige Elimination von Intronsequenzen.

Das veränderte Spleißen eines Gens führt zu einer veränderten Transkriptsequenz (Spleißvariante). Wird eine Spleißvariante im Bereich ihrer veränderten Sequenz translatiert, resultiert ein verändertes Protein, welches sich von dem ursprünglichen in Struktur und Funktion deutlich unterscheiden kann. Bei tumorassoziierten Spleißvarianten können tumorassoziierte Transkripte und tumorassoziierte Proteine/Antigene entstehen. Diese können als molekulare Marker sowohl zum Nachweis von Tumorzellen als auch zum therapeutischen Targeting von Tumoren genutzt werden. Die Detektion von Tumorzellen z.B. im Blut, Serum, Knochenmark, Sputum, Bronchial-Lavage, Körpersekreten und Gewebsbiopsien kann z.B. nach Extraktion von Nukleinsäuren durch PCR-Amplifikation mit Spleiß variantenspezifischen Oligonukleotiden erfolgen.

Zum Nachweis eignen sich alle Sequenz-abhängigen Detektionssysteme. Neben der PCR sind diese z.B. Genchip-/Microarraysysteme, Northern-Blot, RNAse protection assays (RDA) und andere. Allen Detektionssystemen ist gemeinsam, dass die Detektion auf einer spezifischen Hybridisierung mit mindestens einer Spleißvarianten-spezifischen Nukleinsäuresequenz basiert. Die Detektion von Tumorzellen kann jedoch auch durch Antikörper erfolgen, die ein durch die Spleißvariante kodiertes spezifisches Epitop erkennen. Für die Herstellung der Antikörper können Peptide zur Immunisierung verwendet werden, die für diese Spleißvariante spezifisch sind. Die Detektion von Tumorzellen kann auch durch Antikörper erfolgen, die tumorspezifisch veränderte Glykosylierungsvarianten erkennen. Für die Generierung von derartigen Antikörpern können Peptidregionen genutzt werden, die sich in Tumorzellen und gesunden Zellen glykosylierungsbedingt unterscheiden. Durch endogene Deglykosylierung von N-gekoppelten Zuckerresten wird Asparagin in Asparaginsäure transformiert. Daher können die hier beschriebenen Proteine tumorspezifisch sequenzverändert sein und damit andere biochemische und Antikörper-Bindungseigenschaften aufweisen. Für die Immunisierung eignen sich besonders die Aminosäuren, die deutliche Epitopunterschiede zu der/den Variante(n) des Genprodukts aufweisen, welche bevorzugt in gesunden Zellen gebildet wird/werden. Der Nachweis der Tumorzellen mit Antikörper kann dabei an einer vom Patienten isolierten Probe oder als Imaging mit intravenös applizierten Antikörpern erfolgen.

Neben der diagnostischen Nutzbarkeit stellen Spleißvarianten, die neue oder veränderte Epitope aufweisen, attraktive Targets für die Immuntherapie dar. Die hierin offenbarten Epitope können zum Targeting von therapeutisch wirksamen monoklonalen Antikörpern oder T-Lymphozyten genutzt werden. Bei der passiven Immuntherapie werden hierbei Antikörper oder T-Lymphozyten adoptiv transferiert, die Spleißvarianten-spezifische Epitope erkennen. Die Generierung von Antikörpern kann wie bei anderen Antigenen auch unter Nutzung von Standardtechnologien (Immunisierung von Tieren, Panningstrategien zur Isolation von rekombinanten Antikörpern) unter Nutzung von Polypeptiden, die diese Epitope beinhalten, erfolgen. Alternativ können zur Immunisierung Nukleinsäuren genutzt werden, die für Oligo-oder Polypeptide kodieren, die diese Epitope beinhalten. Verschiedene Techniken zur in vitro oder in vivo Generierung von epitopspezifischen T-Lymphozyten sind bekannt und ausführlich beschrieben z.B. (Kessler JH, et al. 2001, Sahin et al., 1997) und basieren ebenfalls auf der Nutzung von Oligo- oder Polypeptiden, die die Spleißvarianten-spezifischen Epitope beinhalten, oder Nukleinsäuren, die für diese kodieren. Oligo- oder Polypeptide, die die Spleißvarianten-spezifischen Epitope beinhalten, oder Nukleinsäuren, die für diese Polypeptide kodieren, sind auch für die Nutzung als pharmazeutisch wirksame Substanzen bei der aktiven Immuntherapie (Vakzinierung, Vakzintherapie) verwendbar.

Die aberrante Expression von Genen in Tumorzellen kann auf veränderte Methylierungsmuster ihrer Promotoren beruhen (De Smet C. et al., Mol. Cell Biol. 24:4781-90, 2004; De Smet C., et al., Mol. Cell Biol. 19:7327-35, 1999; De Smet C. et al., Proc. Natl. Acad. Sei. USA. 93:7149-53, 1996). Diese Methylierungsunterschiede können als indirekter Marker für den im Tumor veränderten Zustand des entsprechenden Gens genutzt werden. Dementsprechend kann die Zunahme oder Abnahme von Basenmethylierungen im Promotorbereich für diagnostische Zwecke genutzt werden.

In einem weiteren Aspekt werden auch posttranslational modifizierte Proteindomänen offenbart wie zum Beispiel Glykosylierungen oder Myristylierungen. Diese Art von Modifikationen kann zu einem differentiellen Erkennungsmuster eines Antigens durch z.B. einen Antikörper führen und verschiedene, unter Umständen krankheitsassoziierte Zustände erkennen. Vor allem durch den Einsatz von Antikörpern kann diese Differenzierung eines Antigens sowohl diagnostisch als auch therapeutisch genutzt werden. Für Tumorzellen ist publiziert, dass die tumorassoziierte zelluläre Entartung zu veränderten posttranslationalen Modifikationen führen kann (Durand & Seta, Clin. Chem. 46:795-8052000; Granovsky et al., Nat. Med. 6:306-312, 2000). Insbesondere Glykosylierungsmuster sind auf Tumorzellen stark verändert. Diese speziellen Epitope können diagnostisch zur Unterscheidung von Tumorzellen und nicht karzinogenen Zellen herangezogen werden. Sollte ein posttranslational modifizierbares Epitop in normalen, nicht entarteten Zellen glykosyliert und in Tumorzellen deglykosyliert sein, erlaubt diese Situation die Entwicklung eines tumorspezifischen therapeutischen Antikörpers im Sinne der Erfindung.

Die Analyse von Proteinmodifikationen kann im Western-Blot erfolgen. Vor allem Glykosylierungen, die in der Regel eine Größe von mehreren kDa haben, führen zu einer größeren Gesamtmasse des Zielproteins, die sich in der SDS-PAGE auftrennen lässt. Zum Nachweis von spezifischen O- und N-glykosidischen Bindungen werden Proteinlysate vor der Denaturierung durch SDS mit O- oder N-Glykosylasen inkubiert (nach Angaben des jeweiligen Herstellers, z.B. PNgase, Endoglykosidase F, Endoglykosidase H, Roche Diagnostics). Anschließend erfolgt ein Western Blot. Bei Verringerung der Größe eines Zielproteins kann so nach Inkubation mit einer Glykosidase eine spezifische Glykosylierung nachgewiesen und auf diesem Weg auch die Tumorspezifität einer Modifikation analysiert werden. Von besonderem Interesse sind Proteinbereiche, die in Tumorzellen und gesunden Zellen differenziell glykosyliert sind. Derartige Glykosylierungsunterschiede sind jedoch bisher für wenige Zelloberflächenproteine (z.B. Muc1) beschrieben.

In einem Aspekt wird eine pharmazeutische Zusammensetzung beschrieben, umfassend ein Mittel, das das gemäß der hierin beschriebenen Strategie identifizierte Tumor-assoziierte Antigen erkennt und vorzugsweise selektiv für Zellen ist, die eine Expression oder abnormale Expression eines gemäß der hierin beschriebenen Strategie identifizierten Tumor-assoziierten Antigens aufweisen. Das Mittel kann in bestimmten Ausführungsformen die Induktion des Zelltods, die Reduktion des Zellwachstums, die Schädigung der Zellmembran oder die Sekretion von Zytokinen bewirken und weist vorzugsweise eine tumorhemmende Aktivität auf. In einer Ausführungsform ist das Mittel eine Antisense-Nukleinsäure, die selektiv mit der Nukleinsäure hybridisiert, die für das Tumor-assoziierte Antigen kodiert. In einer weiteren Ausführungsform ist das Mittel ein Antikörper, der selektiv an das Tumor-assoziierte Antigen bindet, insbesondere ein komplementaktivierter Antikörper oder Toxin-konjugierter Antikörper, der selektiv an das Tumor-assoziierte Antigen bindet. In einer weiteren Ausführungsform umfasst das Mittel mehrere Mittel, die jeweils selektiv verschiedene Tumor-assoziierte Antigene erkennen, wobei mindestens eines der Tumor-assoziierten Antigene ein gemäß der hierin beschriebenen Strategie identifiziertes Tumor-assoziiertes Antigen ist. Die Erkennung muss nicht direkt mit einer Hemmung von Aktivität oder Expression des Antigens einhergehen. In diesem Aspekt der Offenbarung dient das selektiv auf Tumoren beschränkte Antigen vorzugsweise als Markierung zur Rekrutierung von Effektormechanismen an diesen spezifischen Ort. In einer bevorzugten Ausführungsform ist das Mittel ein cytotoxischer T-Lymphozyt, der das Antigen auf einem HLA-Molekül erkennt und die derartig markierte Zelle lysiert. In einer weiteren Ausführungsform ist das Mittel ein Antikörper, der selektiv an das Tumor-assoziierte Antigen bindet und somit natürliche oder artifizielle Effektormechanismen zu dieser Zelle rekrutiert. In einer weiteren Ausführungsform ist das Mittel ein Helfer-T-Lymphozyt, der Effektorfunktionen von anderen Zellen, die spezifisch dieses Antigen erkennen, stärkt.

In einem Aspekt wird eine pharmazeutische Zusammensetzung beschrieben, umfassend ein Mittel, das die Expression oder Aktivität eines gemäß der hierin beschriebenen Strategie identifizierten Tumor-assoziierten Antigens hemmt. In einer bevorzugten Ausführungsform ist das Mittel eine Antisense-Nukleinsäure, die selektiv mit der Nukleinsäure hybridisiert, die für das Tumor-assoziierte Antigen kodiert. In einer weiteren Ausführungsform ist das Mittel ein Antikörper, der selektiv an das Tumor-assoziierte Antigen bindet. In einer weiteren Ausführungsform umfasst das Mittel mehrere Mittel, die jeweils selektiv die Expression oder Aktivität verschiedener Tumor-assoziierter Antigene hemmen, wobei mindestens eines der Tumor-assoziierten Antigene ein gemäß der hierin beschriebenen Strategie identifiziertes Tumor-assoziiertes Antigen ist.

Die Aktivität eines gemäß der hierin beschriebenen Strategie identifizierten Tumor-assoziierten Antigens kann eine jegliche Aktivität eines Proteins oder Peptids sein. Somit können Therapie- und Diagnoseverfahren auch auf Hemmung oder Reduktion dieser Aktivität oder auf ein Testen dieser Aktivität abzielen.

Des Weiteren wird eine pharmazeutische Zusammensetzung beschrieben, die ein Mittel umfasst, das bei einer Verabreichung selektiv die Menge an Komplexen zwischen einem HLA-Molekül und einem Peptidepitop aus dem gemäß der hierin beschriebenen Strategie identifizierten Tumor-assoziierten Antigen erhöht. Das Mittel umfasst in einer Ausführungsform einen oder mehrere Bestandteile, die aus der Gruppe ausgewählt sind bestehend aus (i) dem Tumor-assoziierten Antigen oder einem Teil davon, (ii) einer Nukleinsäure, die für das Tumor-assoziierte Antigen oder einen Teil davon kodiert, (iii) einer Wirtszelle, die das Tumor-assoziierte Antigen oder einen Teil davon exprimiert, und (iv) isolierten Komplexen zwischen Peptidepitopen aus dem Tumor-assoziierten Antigen und einem MHC-Molekül. In einer Ausführungsform umfasst das Mittel mehrere Mittel, die jeweils selektiv die Menge an Komplexen zwischen MHC-Molekülen und Peptidepitopen verschiedener Tumor-assoziierter Antigene erhöhen, wobei mindestens eines der Tumor-assoziierten Antigene ein gemäß der hierin beschriebenen Strategie identifiziertes Tumor-assoziiertes Antigen ist.

Des Weiteren wird eine pharmazeutische Zusammensetzung beschrieben, die einen oder mehrere Bestandteile umfasst, die aus der Gruppe ausgewählt sind bestehend aus (i) einem gemäß der hierin beschriebenen Strategie identifizierten Tumor-assoziierten Antigen oder einem Teil davon, (ii) einer Nukleinsäure, die für ein gemäß der hierin beschriebenen Strategie identifiziertes Tumor-assoziiertes Antigen oder einen Teil davon kodiert, (iii) einem Antikörper, der an ein gemäß der hierin beschriebenen Strategie identifiziertes Tumor-assoziiertes Antigen oder einen Teil davon bindet, (iv) einer Antisense-Nukleinsäure, die spezifisch mit einer Nukleinsäure, die für ein gemäß der hierin beschriebenen Strategie identifiziertes Tumor-assoziiertes Antigen kodiert, hybridisiert, (v) einer Wirtszelle, die ein gemäß der hierin beschriebenen Strategie identifiziertes Tumor-assoziiertes Antigen oder einen Teil davon exprimiert, und (vi) isolierten Komplexen zwischen einem gemäß der hierin beschriebenen Strategie identifizierten Tumor-assoziierten Antigen oder einem Teil davon und einem HLA-Molekül.

Eine Nukleinsäure, die für ein gemäß der hierin beschriebenen Strategie identifiziertes Tumor-assoziiertes Antigen oder einen Teil davon kodiert, kann in der pharmazeutischen Zusammensetzung in einem Expressionsvektor vorliegen und funktionell mit einem Promotor verbunden sein.

Eine in einer pharmazeutischen Zusammensetzung enthaltene Wirtszelle kann das Tumor-assoziierte Antigen oder den Teil davon sekretieren, auf der Oberfläche exprimieren oder kann zusätzlich ein HLA-Molekül exprimieren, das an das Tumor-assoziierte Antigen oder den Teil davon bindet. In einer Ausführungsform exprimiert die Wirtszelle das HLA-Molekül endogen. In einer weiteren Ausführungsform exprimiert die Wirtszelle das HLA-Molekül und/oder das Tumor-assoziierte Antigen oder den Teil davon rekombinant. Vorzugsweise ist die Wirtszelle nicht proliferativ. In einer bevorzugten Ausführungsform ist die Wirtszelle eine Antigen-präsentierende Zelle, insbesondere eine dendritische Zelle, ein Monozyt oder ein Makrophage.

Ein in einer pharmazeutischen Zusammensetzung enthaltener Antikörper kann ein monoklonaler Antikörper sein. In weiteren Ausführungsformen ist der Antikörper ein chimärer oder humanisierter Antikörper, ein Fragment eines natürlichen Antikörpers oder ein synthetischer Antikörper, die durch kombinatorische Techniken hergestellt werden können. Der Antikörper kann mit einem therapeutisch oder diagnostisch nützlichen Mittel gekoppelt sein.

Eine in einer pharmazeutischen Zusammensetzung enthaltene Antisense-Nukleinsäure kann eine Sequenz von 6-50, insbesondere 10-30, 15-30 oder 20-30 zusammenhängenden Nukleotiden aus der Nukleinsäure, die für das gemäß der hierin beschriebenen Strategie identifizierte Tumor-assoziierte Antigen kodiert, umfassen.

In weiteren Ausführungsformen bindet ein durch eine pharmazeutische Zusammensetzung entweder direkt oder durch die Expression einer Nukleinsäure bereitgestelltes Tumor-assoziiertes Antigen oder ein Teil davon an MHC-Moleküle auf der Oberfläche von Zellen, wobei die Bindung vorzugsweise eine cytolytische Reaktion hervorruft und/oder eine Zytokinausschüttung induziert.

Eine pharmazeutische Zusammensetzung kann einen pharmazeutisch verträglichen Träger und/oder ein Adjuvans umfassen. Das Adjuvans kann aus Saponin, GM-CSF, CpG-Nukleotiden, RNA, einem Zytokin oder einem Chemokin ausgewählt sein. Eine pharmazeutische Zusammensetzung wird vorzugsweise zur Behandlung einer Erkrankung eingesetzt, die sich durch die selektive Expression oder abnormale Expression eines Tumor-assoziierten Antigens auszeichnet. In einer bevorzugten Ausführungsform ist die Erkrankung Krebs.

Die Erfindung betrifft Verfahren zur Diagnose oder Überwachung, d.h. Bestimmung der Regression, des Verlaufs und/oder des Ausbruchs, einer Krebserkrankung, die sich durch die Expression oder abnormale Expression eines oder mehrerer Tumor-assoziierter Antigene auszeichnet, wie in den Patentansprüchen definiert, und offenbart Behandlungsverfahren für eine solche Erkrankung.

In einer Ausführungsform umfasst die Behandlung die Verabreichung einer pharmazeutischen Zusammensetzung.

Die erfindungsgemäßen Diagnoseverfahren und/oder Verfahren zur Überwachung betreffen allgemein die Verwendung von Mitteln für den Nachweis und/oder die Bestimmung bzw. Überwachung der Menge (i) einer Nukleinsäure, die für das Tumor-assoziierte Antigen kodiert, oder eines Teils davon und/oder (ii) des Tumor-assoziierten Antigens oder eines Teils davon, in einer aus einem Patienten isolierten biologischen Probe.

In einem Aspekt betrifft die Erfindung ein Verfahren zur Diagnose einer Krebserkrankung, die sich durch die Expression oder abnormale Expression eines gemäß der hierin beschriebenen Strategie identifizierten Tumor-assoziierten Antigens auszeichnet, wie im Patentanspruch 1 definiert. Das Verfahren umfasst den Nachweis (i) einer Nukleinsäure, die für das Tumor-assoziierte Antigen kodiert, oder eines Teils davon und/oder (ii) den Nachweis des Tumor-assoziierten Antigens oder eines Teils davon. In bestimmten Ausführungsformen umfasst der Nachweis (i) die Kontaktierung der biologischen Probe mit einem Mittel, das spezifisch an die Nukleinsäure, die für das Tumor-assoziierte Antigen kodiert, oder den Teil davon oder an das Tumor-assoziierte Antigen oder den Teil davon bindet und (ii) den Nachweis der Komplexbildung zwischen dem Mittel und der Nukleinsäure oder dem Teil davon oder dem Tumor-assoziierten Antigen oder dem Teil davon. In einer Ausführungsform zeichnet sich die Erkrankung durch die Expression oder abnormale Expression mehrerer verschiedener Tumor-assoziierter Antigene aus und der Nachweis umfasst einen Nachweis mehrerer Nukleinsäuren, die für die mehreren verschiedenen Tumor-assoziierten Antigene kodieren, oder von Teilen davon oder den Nachweis der mehreren verschiedenen Tumor-assoziierten Antigene oder von Teilen davon. In einer weiteren Ausführungsform wird die isolierte biologische Probe aus dem Patienten mit einer vergleichbaren normalen biologischen Probe verglichen.

Diagnoseverfahren können auch veränderte Methylierungsmuster des Promotorbereiches des jeweiligen Tumor-assoziierten Genproduktes nutzen. Der Nachweis solcher Methylierungsmuster kann unter Nutzung von auf PCR basierenden Verfahren, mit Hilfe von Restriktionsenzymen oder durch Sequenzierung erfolgen. Ein dazu geeignetes Test kann dabei folgendermaßen aufgebaut sein: (1) Extraktion von DNA aus Gewebeproben von Patienten z.B. unter Nutzung von paraffineingebettetem Material, (2) Behandlung der DNA mit Bisulfit-haltigen Reagenzien (z.B. wie beschrieben in Clark SJ et al., Nucleic Acids Res. 22(15):2990-7, 1994), (3) Amplifikation von DNA mit PCR, und (4) Analyse durch Bestimmung der Menge sequenzspezifischer Amplifikationsprodukte (z.B. durch quantitative PCR, Hybridisierungsverfahren wie Microarrayverfahren).

Die erfindungsgemäßen Diagnoseverfahren können auch eine Nutzung der gemäß der hierin beschriebenen Strategie identifizierten Tumor-assoziierten Antigene als prognostische Marker betreffen, um eine Metastasierung z.B. durch Testen des Migrationsverhalten von Zellen und daher einen verschlechterten Krankheitsverlauf zu prädizieren, wodurch unter anderem die Planung einer aggressiveren Therapie ermöglicht wird.

In einem weiteren Aspekt betrifft die Erfindung ein Verfahren zur Bestimmung der Regression, des Verlaufs oder des Ausbruchs einer Krebserkrankung, die sich durch die Expression oder abnormale Expression eines gemäß der hierin beschriebenen Strategie identifizierten Tumor-assoziierten Antigens auszeichnet, umfassend die Überwachung einer Probe aus einem Patienten, der die Erkrankung aufweist oder in Verdacht steht, an der Erkrankung zu erkranken in Bezug auf einen oder mehrere Parameter, die aus der Gruppe ausgewählt sind bestehend aus (i) der Menge der Nukleinsäure, die für das Tumor-assoziierte Antigen kodiert, oder eines Teil davon und (ii) der Menge des Tumor-assoziierten Antigens oder eines Teils davon, wie im Patentanspruch 2 definiert. Vorzugsweise umfasst das Verfahren die Bestimmung des oder der Parameter zu einem ersten Zeitpunkt in einer ersten Probe und zu einem zweiten Zeitpunkt in einer weiteren Probe, wobei durch einen Vergleich der beiden Proben der Verlauf der Erkrankung ermittelt wird. In bestimmten Ausführungsformen zeichnet sich die Erkrankung durch die Expression oder abnormale Expression mehrerer verschiedener Tumor-assoziierter Antigene aus und die Überwachung umfasst eine Überwachung (i) der Menge mehrerer Nukleinsäuren, die für die mehreren verschiedenen Tumor-assoziierten Antigene kodieren, oder von Teilen davon und/oder (ii) der Menge der mehreren verschiedenen Tumor-assoziierten Antigene oder von Teilen davon.

Ein Nachweis einer Nukleinsäure oder eines Teils davon oder eine Bestimmung bzw. Überwachung der Menge einer Nukleinsäure oder eines Teils davon kann erfindungsgemäß mit einer Polynukleotid-Sonde erfolgen, die spezifisch mit der Nukleinsäure oder dem Teil davon hybridisiert, oder kann durch selektive Amplifikation der Nukleinsäure oder des Teils davon erfolgen. In einer Ausführungsform umfasst die Polynukleotid-Sonde eine Sequenz von 6-50, insbesondere 10-30, 15-30 oder 20-30 zusammenhängenden Nukleotiden aus der Nukleinsäure.

In bestimmten Ausführungsformen der erfindungsgemäßen Diagnoseverfahren erfolgt eine selektive Amplifikation des Promotorbereichs oder eines Teils davon einer Nukleinsäure, die für ein gemäß der hierin beschriebenen Strategie identifiziertes Tumor-assoziiertes Antigen kodiert und in Form von genomischer DNA vorliegt, nach Behandlung mit einem bisulfithaltigen Reagenz. Die Nukleinsäure wird vorzugsweise vor einer Behandlung mit dem bisulfithaltigen Reagenz aus einer Probe eines zu untersuchenden Patienten isoliert. Die bei einer solchen Amplifikation verwendeten Oligonukleotide weisen vorzugsweise eine Sequenz auf, die an die mit dem bisulfithaltigen Reagenz behandelte Nukleinsäure binden, vorzugsweise dazu vollständig komplementär sind. Vorzugsweise sind die Oligonukleotide einem unterschiedlichen Grad einer Methylierung der Nukleinsäure angepasst und bedingen differenzierbare Amplifikationsprodukte.

Ein Nachweis eines Tumor-assoziierten Antigens oder eines Teils davon oder eine Bestimmung bzw. Überwachung der Menge eines Tumor-assoziierten Antigens oder eines Teils davon erfolgt erfindungsgemäß mit einem Antikörper, der spezifisch an das Tumor-assoziierte Antigen oder den Teil davon bindet.

In bestimmten Ausführungsformen liegt das nachzuweisende Tumor-assoziierte Antigen oder der Teil davon in einem Komplex mit einem MHC-Molekül, insbesondere einem HLA-Molekül vor.

Die für einen Nachweis oder für eine Bestimmung bzw. Überwachung verwendete Polynukleotid-Sonde oder der Antikörper ist vorzugsweise nachweisbar markiert. In bestimmten Ausführungsformen ist der nachweisbare Marker ein radioaktiver Marker oder ein Enzymmarker.

Ein Verfahren zur Behandlung, Diagnose oder Überwachung einer Erkrankung, die sich durch die Expression oder abnormale Expression eines gemäß der hierin beschriebenen Strategie identifizierten Tumor-assoziierten Antigens auszeichnet, kann die Verabreichung eines Antikörpers umfassen, der an das Tumor-assoziierte Antigen oder einen Teil davon bindet und mit einem therapeutischen oder diagnostischen Mittel oder Stoff gekoppelt ist. Der Antikörper kann ein monoklonaler Antikörper sein. In weiteren Ausführungsformen ist der Antikörper ein chimärer oder humanisierter Antikörper oder ein Fragment eines natürlichen Antikörpers.

In einer weiteren Ausführungsform werden Oligonukleotide offenbart, die mit einer gemäß der hierin beschriebenen Strategie identifizierten Nukleinsäure hybridisieren und als genetische Sonden verwendet werden können. Nukleinsäuremoleküle in der Form von Oligonukleotid-Primern oder kompetenten Proben, die mit einer gemäß der hierin beschriebenen Strategie identifizierten Nukleinsäure oder Teilen davon hybridisieren, können zum Auffinden von Nukleinsäuren verwendet werden, die zu der gemäß der hierin beschriebenen Strategie identifizierten Nukleinsäure homolog sind. PCR-Amplifikation, Southern- und Northern-Hybridisierung können zum Auffinden homologer Nukleinsäuren eingesetzt werden. Die Hybridisierung kann unter niedrig-, besser unter mittel- und am besten unter hoch-stringenten Bedingungen erfolgen. Der Begriff "stringente Bedingungen", wie hierin verwendet, betrifft Bedingungen, die eine spezifische Hybridisierung zwischen Polynukleotiden erlauben.

In einem weiteren Aspekt wird ein Protein, Polypeptid oder Peptid offenbart, das von einer Nukleinsäure kodiert wird, die aus der Gruppe ausgewählt ist bestehend aus (a) einer Nukleinsäure, die eine Nukleinsäuresequenz umfasst, die aus der Gruppe bestehend aus SEQ ID NO: 5 des Sequenzprotokollseinem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist, und (d) einer Nukleinsäure, die zu der Nukleinsäure unter (a), (b) oder (c) komplementär ist. In einer bevorzugten Ausführungsform wird ein Protein oder Polypeptid offenbart, das eine Aminosäuresequenz umfasst, ausgewählt aus der Gruppe bestehend aus SEQ ID NOs: 6, 282 und 283 des Sequenzprotokolls, einem Teil oder Derivat davon.

In einem weiteren Aspekt wird ein immunogenes Fragment eines gemäß der hierin beschriebenen Strategie identifizierten Tumor-assoziierten Antigens offenbart. Das Fragment bindet vorzugsweise an einen menschlichen HLA-Rezeptor oder menschlichen Antikörper. Vorzugsweise umfasst ein Fragment eine Sequenz von mindestens 6, insbesondere mindestens 8, mindestens 10, mindestens 12, mindestens 15, mindestens 20, mindestens 30 oder mindestens 50 Aminosäuren.

In diesem Aspekt wird insbesondere ein Peptid offenbart, das eine Sequenz gemäß SEQ ID NO: 282 oder 283 aufweist oder umfasst.

In einem weiteren Aspekt wird ein Mittel offenbart, das an ein gemäß der hierin beschriebenen Strategie identifiziertes Tumor-assoziiertes Antigen oder an einen Teil davon bindet. In einer bevorzugten Ausführungsform ist das Mittel ein Antikörper. In weiteren Ausführungsformen ist der Antikörper ein chimärer, ein humanisierter oder mit kombinatorischen Techniken hergestellte Antikörper oder ein Fragment eines Antikörpers. Des Weiteren wird ein Antikörper offenbart, der selektiv an einen Komplex aus (i) einem gemäß der hierin beschriebenen Strategie identifizierten Tumor-assoziierten Antigen oder einem Teil davon und (ii) einem MHC-Molekül bindet, an das das gemäß der hierin beschriebenen Strategie identifizierte Tumor-assoziierte Antigen oder der Teil davon bindet, wobei der Antiköper nicht alleine an (i) oder (ii) bindet. Ein hierin beschriebener Antikörper kann ein monoklonaler Antikörper sein. In weiteren Ausführungsformen ist der Antikörper ein chimärer oder humanisierter Antikörper oder ein Fragment eines natürlichen Antikörpers.

Des Weiteren wird ein Konjugat zwischen einem hierin beschriebenen Mittel, das an ein gemäß der hierin beschriebenen Strategie identifiziertes Tumor-assoziiertes Antigen oder an einen Teil davon bindet, oder einem hierin beschriebenen Antikörper und einem diagnostischen Mittel oder Stoff offenbart. In einer Ausführungsform ist das diagnostische Mittel ein Toxin.

In einem weiteren Aspekt betrifft die Erfindung die Verwendung eines Kits zur Diagnose einer Krebserkrankung, die sich durch die Expression oder abnormalen Expression eines gemäß der hierin beschriebenen Strategie identifizierten Tumor-assoziierten Antigens auszeichnet, umfassend Mittel zum Nachweis (i) der Nukleinsäure, die für das Tumor-assoziierte Antigen kodiert, oder eines Teils davon, oder (ii) des Tumor-assoziierten Antigens oder eines Teils davon, wobei das Mittel ein Antikörper ist, der für das Tumor-assoziierte Antigen spezifisch ist, wie im Patentanspruch 6 definiert. In einer Ausführungsform sind die Mittel zum Nachweis der Nukleinsäure oder des Teils davon Nukleinsäuremoleküle für die selektive Amplifikation der Nukleinsäure, die insbesondere eine Sequenz von 6-50, insbesondere 10-30, 15-30 oder 20-30 zusammenhängenden Nukleotiden aus der Nukleinsäure umfassen.

Die Erfindung betrifft insbesondere ein Verfahren zur Diagnose einer Krebserkrankung, die sich durch die Expression oder abnormale Expression eines Tumor-assoziierten Antigens auszeichnet, umfassend
(i) den Nachweis einer Nukleinsäure, die für das Tumor-assoziierte Antigen kodiert, in einer aus einem Patienten isolierten biologischen Probe, und/oder
(ii) den Nachweis des Tumor-assoziierten Antigens in einer aus einem Patienten isolierten biologischen Probe,
   wobei das Verfahren gemäß (ii) die Kontaktierung der biologischen Probe mit einem Antikörper und den Nachweis der Komplexbildung zwischen dem Antikörper und dem Tumor-assoziierten Antigen umfasst, wobei der Antikörper spezifisch an das Tumor-assoziierte Antigen bindet,
   wobei das Tumor-assoziierte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz gemäß SEQ ID NO: 5 des Sequenzprotokolls umfasst, und
   (b) einer Nukleinsäure, die zu der Nukleinsäure unter (a) mindestens 90% Sequenzidentität aufweist.

Die vorliegende Erfindung betrifft weiterhin ein Verfahren zur Bestimmung der Regression, des Verlaufs oder des Ausbruchs einer Krebserkrankung, die sich durch die Expression oder abnormale Expression eines Tumor-assoziierten Antigens auszeichnet, umfassend die Überwachung einer Probe aus einem Patienten, der die Erkrankung aufweist oder in Verdacht steht, an der Erkrankung zu erkranken, in Bezug auf einen oder mehrere Parameter, ausgewählt aus der Gruppe, bestehend aus:
(i) der Menge der Nukleinsäure, die für das Tumor-assoziierte Antigen kodiert, und
(ii) der Menge des Tumor-assoziierten Antigens,
   wobei das Verfahren gemäß (ii) die Kontaktierung der biologischen Probe mit einem Antikörper und den Nachweis der Komplexbildung zwischen dem Antikörper und dem Tumor-assoziierten Antigen umfasst, wobei der Antikörper spezifisch an das Tumor-assoziierte Antigen bindet,
   wobei das Tumor-assoziierte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz gemäß SEQ ID NO: 5 des Sequenzprotokolls umfasst, und
   (b) einer Nukleinsäure, die zu der Nukleinsäure unter (a) mindestens 90% Sequenzidentität aufweist.

In einer Ausführungsform ist der Antikörper ein monoklonaler, chimärer oder humanisierter Antikörper oder ein Fragment eines Antikörpers.

In einer weiteren Ausführungsform ist die Erkrankung ein Nierenkrebs, ein Brustkrebs, ein Pankreaskrebs, ein Lungenkarzinom, ein Magenkarzinom oder ein Mammakarzinom.

In einer weiteren Ausführungsform umfasst das Tumor-assoziierte Antigen eine Aminosäuresequenz gemäß SEQ ID NO: 6 des Sequenzprotokolls.

Die vorliegende Erfindung betrifft weiterhin die Verwendung eines Kits zur Diagnose einer Krebserkrankung, die sich durch die Expression oder abnormale Expression eines Tumor-assoziierten Antigens auszeichnet, umfassend Mittel zum Nachweis
(i) der Nukleinsäure, die für das Tumor-assoziierte Antigen kodiert, oder
(ii) des Tumor-assoziierten Antigens,
   wobei die Diagnose gemäß (ii) die Kontaktierung einer biologischen Probe mit dem Mittel und den Nachweis der Komplexbildung zwischen dem Mittel und dem Tumor-assoziierten Antigen umfasst, wobei das Mittel ein Antikörper ist, der spezifisch an das Tumor-assoziierte Antigen bindet,
   wobei das Tumor-assoziierte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz gemäß SEQ ID NO: 5 des Sequenzprotokolls umfasst, und
   (b) einer Nukleinsäure, die zu der Nukleinsäure unter (a) mindestens 90% Sequenzidentität aufweist.

### Detaillierte Beschreibung der Erfindung

Hierin wird ein Gen beschrieben, das in Tumorzellen selektiv oder aberrant exprimiert wird und ein Tumor-assoziiertes Antigen darstellt. Solche Gene und/oder deren Genprodukte und/oder ihre Derivate und/oder Teile sind bevorzugte Zielstrukturen für therapeutische Ansätze. Konzeptionell können die therapeutischen Ansätze auf eine Hemmung der Aktivität des selektiv exprimierten Tumor-assoziierten Genproduktes zielen. Dies ist dann sinnvoll, wenn die aberrante respektive selektive Expression funktionell von tumorpathogenetischer Bedeutung ist und ihre Unterbindung mit einer selektiven Schädigung der entsprechenden Zellen einhergeht. Andere therapeutische Konzepte betrachten Tumor-assoziierte Antigene als Markierungen, die Effektormechanismen mit zellschädigendem Potential selektiv zu Tumorzellen rekrutieren. Hierbei ist die Funktion des Zielmoleküls selbst und seine Rolle bei der Tumorentstehung vollkommen unerheblich.

Mit "Derivat" einer Nukleinsäure, wie hierin verwendet, ist gemeint, dass einzelne oder multiple Nukleotidsubstitutionen, -deletionen und/oder -additionen in der Nukleinsäure vorliegen. Weiterhin umfasst der Begriff "Derivat" auch eine chemische Derivatisierung einer Nukleinsäure an einer Nukleotidbase, am Zucker oder am Phosphat eines Nukleotids. Der Begriff "Derivat" umfasst auch Nukleinsäuren, die nicht in der Natur vorkommende Nukleotide und Nukleotidanaloga enthalten.

Eine Nukleinsäure ist vorzugsweise Desoxyribonukleinsäure (DNA) oder Ribonukleinsäure (RNA). Nukleinsäuren umfassen genomische DNA, cDNA, mRNA, rekombinant hergestellte und chemisch synthetisierte Moleküle. Eine Nukleinsäure kann als einzelsträngiges oder doppelsträngiges und lineares oder kovalent kreisförmig geschlossenes Molekül vorliegen.

Die hierin beschriebenen Nukleinsäuren sind vorzugsweise isoliert. Der Begriff "isolierte Nukleinsäure", wie hierin verwendet, bedeutet, dass die Nukleinsäure (i) *in vitro* amplifiziert wurde, zum Beispiel durch Polymerase-Kettenreaktion (PCR), (ii) rekombinant durch Klonierung produziert wurde, (iii) gereinigt wurde, zum Beispiel durch Spaltung und gelelektrophoretische Auftrennung, oder (iv) synthetisiert wurde, zum Beispiel durch chemische Synthese. Eine isolierte Nukleinsäure ist eine Nukleinsäure, die für eine Manipulierung durch rekombinante DNA-Techniken zur Verfügung steht.

Eine Nukleinsäure ist dann zu einer anderen Nukleinsäure "komplementär", wenn die beiden Sequenzen miteinander hybridisieren und ein stabiles Duplexmolekül eingehen können, wobei die Hybridisierung vorzugsweise unter Bedingungen erfolgt, die eine spezifische Hybridisierung zwischen Polynukleotiden erlauben (stringente Bedingungen). Stringente Bedingungen sind beispielsweise in Molecular Cloning: A Laboratory Manual, J. Sambrook et al., Hrsg., 2. Auflage, Cold Spring Harbor Laboratory Press, Cold Spring Harbor, New York, 1989, oder Current Protocols in Molecular Biology, F.M. Ausubel et al., Hrsg., John Wiley & Sons, Inc., New York beschrieben und betreffen beispielsweise die Hybridisierung bei 65°C in Hybridisierungspuffer (3,5 x SSC, 0,02% Ficoll, 0,02% Polyvinylpyrrolidon, 0,02% Rinderserumalbumin, 2,5 mM NaH₂PO₄ (pH 7), 0,5% SDS, 2 mM EDTA). SSC ist eine Lösung mit jeweils 0,15 M Natriumchlorid und Natriumcitrat, pH 7. Nach der Hybridisierung wird die Membran, auf die die DNA übertragen wurde, beispielsweise in 2 x SSC bei Raumtemperatur und sodann in 0,1 - 0,5 x SSC / 0,1 x SDS bei Temperaturen bis 68°C gewaschen.

Komplementäre Nukleinsäuren weisen mindestens 40%, insbesondere mindestens 50%, mindestens 60%, mindestens 70%, mindestens 80%, mindestens 90% und vorzugsweise mindestens 95%, mindestens 98% oder mindestens 99% Identität der Nukleotide auf.

Nukleinsäuren, die für Tumor-assoziierte Antigene kodieren, können alleine oder in Kombination mit anderen Nukleinsäuren, insbesondere heterologen Nukleinsäuren, vorliegen. In bevorzugten Ausführungsformen liegt eine Nukleinsäure funktionell in Verbindung mit Expressionskontrollsequenzen oder regulatorischen Sequenzen vor, die in Bezug zu der Nukleinsäure homolog oder heterolog sein können. Eine kodierende Sequenz und eine regulatorische Sequenz sind dann "funktionell" miteinander verbunden, falls sie derart kovalent miteinander verknüpft sind, dass die Expression oder Transkription der kodierenden Sequenz unter der Kontrolle oder unter dem Einfluss der regulatorischen Sequenz steht. Falls die kodierende Sequenz in ein funktionelles Protein translatiert werden soll, führt bei einer funktionellen Verbindung einer regulatorischen Sequenz mit der kodierenden Sequenz eine Induktion der regulatorischen Sequenz zu einer Transkription der kodierenden Sequenz, ohne dass es zu einer Leserasterverschiebung in der kodierenden Sequenz oder zu einem Unvermögen der kodierenden Sequenz kommt, in das gewünschte Protein oder Peptid translatiert zu werden.

Der Begriff "Expressionskontrollsequenz" oder "regulatorische Sequenz", wie hierin verwendet, umfasst Promotoren, Enhancer und andere Kontrollelemente, die die Expression eines Gens steuern. In bestimmten Ausführungsformen sind die Expressionskontrollsequenzen regulierbar. Die genaue Struktur von regulatorischen Sequenzen kann speziesabhängig oder zelltypusabhängig variieren, umfasst jedoch im Allgemeinen 5'-nicht-transkribierte und 5'-nicht-translatierte Sequenzen, die an der Initiation der Transkription bzw. Translation beteiligt sind wie TATA-Box, Capping-Sequenz, CAAT-Sequenz und ähnliches. Insbesondere umfassen 5'-nicht-transkribierte Regulationssequenzen eine Promotorregion, die eine Promotorsequenz für eine transkriptionelle Kontrolle des funktionell verbundenen Gens einschließt. Regulatorische Sequenzen können auch EnhancerSequenzen oder stromaufwärts gelegene Aktivatorsequenzen umfassen.

Zum einen können also die hier dargestellten Tumor-assoziierten Antigene mit beliebigen Expressionskontrollsequenzen und Promotoren kombiniert werden. Zum anderen aber können die Promotoren der hier dargestellten Tumor-assoziierten Genprodukte mit beliebigen anderen Genen kombiniert werden. Dies erlaubt, die selektive Aktivität dieser Promotoren zu nutzen.

Des Weiteren kann eine Nukleinsäure in Verbindung mit einer anderen Nukleinsäure vorliegen, die für ein Polypeptid kodiert, das eine Sekretion des durch die Nukleinsäure kodierten Proteins oder Polypeptids aus einer Wirtszelle steuert. Auch kann eine Nukleinsäure in Verbindung mit einer anderen Nukleinsäure vorliegen, die für ein Polypeptid kodiert, das eine Verankerung des kodierten Proteins oder Polypeptids auf der Zellmembran der Wirtszelle oder seine Kompartimentalisierung in bestimmte Organellen dieser Zelle herbeiführt. Gleichermaßen kann eine Verbindung mit einer Nukleinsäure erfolgen, die ein Reportergen oder einen beliebigen "Tag" darstellt.

In einer bevorzugten Ausführungsform ist ein rekombinantes DNA-Molekül ein Vektor, gegebenenfalls mit einem Promotor, der die Expression einer Nukleinsäure, z.B. einer Nukleinsäure, die für ein gemäß der hierin beschriebenen Strategie identifiziertes Tumor-assoziiertes Antigen kodiert, steuert. Der Begriff "Vektor" wird dabei in seiner allgemeinsten Bedeutung verwendet und umfasst jegliche intermediären Vehikel für eine Nukleinsäure, die es z.B. ermöglichen, die Nukleinsäure in prokaryontische und/oder in eukaryontische Zellen einzubringen und gegebenenfalls in ein Genom zu integrieren. Solche Vektoren werden vorzugsweise in der Zelle repliziert und/oder exprimiert. Ein intermediäres Vehikel kann z.B. für den Gebrauch bei der Elektroporation, beim Mikroprojektilbeschuss, bei der liposomalen Verabreichung, beim Transfer mit Hilfe von Agrobakterien oder bei der Insertion über DNA-oder RNA-Viren angepasst sein. Vektoren umfassen Plasmide, Phagemide, Bacteriophage oder Virusgenome.

Die Nukleinsäuren, die für ein gemäß der hierin beschriebenen Strategie identifiziertes Tumor-assoziiertes Antigen kodieren, können für eine Transfektion von Wirtszellen eingesetzt werden. Mit Nukleinsäuren ist dabei sowohl rekombinante DNA wie auch RNA gemeint. Rekombinante RNA kann durch *in vitro*-Transkription von einer DNA-Matrize hergestellt werden. Sie kann des Weiteren vor Applikation durch stabilisierende Sequenzen, Capping und Poly-Adenylierung modifiziert werden.

Der Begriff "Wirtszelle", wie hierin verwendet, betrifft jede Zelle, die mit einer exogenen Nukleinsäure transformierbar oder transfizierbar ist. Der Begriff "Wirtszellen", wie hierin verwendet, umfasst prokaryontische (z.B. *E. coli*) oder eukaryontische (z.B. dendritische Zellen, B-Zellen, CHO-Zellen, COS-Zellen, K562-Zellen, Hefezellen und Insektenzellen). Besonders bevorzugt sind Säugerzellen wie Zellen aus Mensch, Maus, Hamster, Schwein, Ziege und Primaten. Die Zellen können aus einer Vielzahl von Gewebetypen abgeleitet sein und umfassen primäre Zellen und Zelllinien. Spezifische Beispiele umfassen Keratinozyten, periphere Blutleukozyten, Stammzellen des Knochenmarks und embryonale Stammzellen. In weiteren Ausführungsformen ist die Wirtszelle eine Antigen-präsentierende Zelle, insbesondere eine dendritische Zelle, ein Monozyt oder Makrophage. Eine Nukleinsäure kann in der Wirtszelle in einer einzigen oder in mehreren Kopien vorliegen und wird in einer Ausführungsform in der Wirtszelle exprimiert.

Der Begriff "Expression" wird hierin in seiner allgemeinsten Bedeutung verwendet und umfasst die Produktion von RNA oder von RNA und Protein. Er umfasst auch eine teilweise Expression von Nukleinsäuren. Des Weiteren kann die Expression transient oder stabil erfolgen. Bevorzugte Expressionssysteme in Säugerzellen umfassen pcDNA3.1 und pRc/CMV (Invitrogen, Carlsbad, CA), die einen selektierbaren Marker enthalten wie ein Gen, das eine Resistenz gegenüber G418 verleiht (und somit eine Selektion stabil transfizierter Zelllinien ermöglicht), und die Enhancer-Promotor-Sequenzen von Cytomegalovirus (CMV).

In den hierin beschriebenen Fällen, in denen ein HLA-Molekül ein Tumor-assoziiertes Antigen oder einen Teil davon präsentiert, kann ein Expressionsvektor auch eine Nukleinsäuresequenz umfassen, die für das HLA-Molekül kodiert. Die Nukleinsäuresequenz, die für das HLA-Molekül kodiert, kann auf demselben Expressionsvektor wie die Nukleinsäure, die für das Tumor-assoziierte Antigen oder den Teil davon kodiert, vorliegen oder beide Nukleinsäuren können auf verschiedenen Expressionsvektoren vorliegen. Im letzteren Fall können die beiden Expressionsvektoren in eine Zelle cotransfiziert werden. Falls eine Wirtszelle weder das Tumor-assoziierte Antigen oder den Teil davon noch das HLA-Molekül exprimiert, werden beide dafür kodierenden Nukleinsäuren entweder auf demselben Expressionsvektor oder auf verschiedenen Expressionsvektoren in die Zelle transfiziert. Falls die Zelle bereits das HLA-Molekül exprimiert, kann nur die Nukleinsäuresequenz, die für das Tumor-assoziierte Antigen oder den Teil davon kodiert, in die Zelle transfiziert werden.

Auch hierin offenbart sind Kits zur Amplifikation einer Nukleinsäure, die für ein Tumor-assoziiertes Antigen kodiert. Solche Kits umfassen beispielsweise ein Paar von Amplifikationsprimern, die an die Nukleinsäure hybridisieren, die für das Tumor-assoziierte Antigen kodiert. Die Primer umfassen vorzugsweise eine Sequenz von 6-50, insbesondere 10-30, 15-30 oder 20-30 zusammenhängenden Nukleotiden aus der Nukleinsäure und sind nicht-überlappend, um die Bildung von Primer-Dimeren zu vermeiden. Einer der Primer wird an einen Strang der Nukleinsäure hybridisieren, die für das Tumor-assoziierte Antigen kodiert, und der andere Primer wird an den komplementären Strang in einer Anordnung hybridisieren, die eine Amplifikation der Nukleinsäure, die für das Tumor-assoziierte Antigen kodiert, erlaubt.

"Antisense"-Moleküle oder "Antisense"-Nukleinsäuren können zur Regulierung, insbesondere der Reduktion der Expression einer Nukleinsäure verwendet werden. Der Begriff "Antisense-Molekül" oder "Antisense-Nukleinsäure", wie hierin verwendet, betrifft ein Oligonukleotid, das ein Oligoribonukleotid, Oligodesoxyribonukleotid, modifiziertes Oligoribonukleotid oder modifiziertes Oligodesoxyribonukleotid ist und das unter physiologischen Bedingungen an DNA, die ein bestimmtes Gen umfasst, oder mRNA dieses Gens hybridisiert, wodurch die Transkription dieses Gens und/oder die Translation dieser mRNA gehemmt wird. Ein "Antisense-Molekül", wie hierin verwendet, umfasst auch ein Konstrukt, das eine Nukleinsäure oder einen Teil davon in reverser Orientierung in Bezug auf ihren natürlichen Promotor enthält. Ein Antisense-Transkript einer Nukleinsäure oder eines Teils davon kann ein Duplexmolekül mit der natürlich vorkommenden mRNA, die das Enzym spezifiziert, eingehen und so eine Akkumulation von oder die Translation der mRNA in das aktive Enzym verhindern. Eine weitere Möglichkeit ist die Verwendung von Ribozymen zur Inaktivierung einer Nukleinsäure. Bevorzugte Antisense-Oligonukleotide weisen eine Sequenz von 6-50, insbesondere 10-30, 15-30 oder 20-30 zusammenhängenden Nukleotiden aus der Ziel-Nukleinsäure auf und sind vorzugsweise vollständig zu der Ziel-Nukleinsäure oder einem Teil davon komplementär.

In bevorzugten Ausführungsformen hybridisiert das Antisense-Oligonukleotid mit einer N-terminalen oder 5'-stromaufwärts gelegenen Stelle wie einer Translationsinitiations-, Transkriptionsinitiations- oder Promotorstelle. In weiteren Ausführungsformen hybridisiert das Antisense-Oligonukleotid mit einer 3'-nicht-translatierten Region oder mRNA-SpleißStelle.

In einer Ausführungsform besteht ein hierin beschriebenes Oligonukleotid aus Ribonukleotiden, Desoxyribonukleotiden oder einer Kombination davon. Dabei sind das 5'-Ende eines Nukleotids und das 3'-Ende eines anderen Nukleotids durch eine Phosphodiesterbindung miteinander verknüpft. Diese Oligonukleotide können in herkömmlicher Weise synthetisiert oder rekombinant produziert werden.

In bevorzugten Ausführungsformen ist ein hierin beschriebenes Oligonukleotid ein "modifiziertes" Oligonukleotid. Dabei kann das Oligonukleotid, um beispielsweise seine Stabilität oder therapeutische Wirksamkeit zu erhöhen, auf verschiedenste Art und Weise modifiziert sein ohne dass seine Fähigkeit, an sein Ziel zu binden, beeinträchtigt wird. Der Begriff "modifiziertes Oligonukleotid", wie hierin verwendet, bedeutet ein Oligonukleotid, bei dem (i) mindestens zwei seiner Nukleotide durch eine synthetische Internukleosidbindung (d.h. eine Internukleosidbindung, die keine Phosphodiesterbindung ist) miteinander verknüpft sind und/oder (ii) eine chemische Gruppe kovalent mit dem Oligonukleotid verbunden ist, die normalerweise nicht bei Nukleinsäuren auftritt. Bevorzugte synthetische Internukleosidbindungen sind Phosphorothioate, Alkylphosphonate, Phosphorodithioate, Phosphatester, Alkylphosphonothioate, Phosphoramidate, Carbamate, Carbonate, Phosphattriester, Acetamidate, Carboxymethylester und Peptide.

Der Begriff "modifiziertes Oligonukleotid" umfasst auch Oligonukleotide mit einer kovalent modifizierten Base und/oder Zucker. "Modifizierte Oligonukleotide" umfassen beispielsweise Oligonukleotide mit Zuckerresten, die kovalent an organische Gruppen mit einem geringen Molekulargewicht gebunden sind, die keine Hydroxylgruppe an der 3'-Position und keine Phosphatgruppe an der 5'-Position sind. Modifizierte Oligonukleotide können beispielsweise einen 2'-O-alkylierten Riboserest oder einen anderen Zucker anstelle von Ribose wie Arabinose umfassen.

Die hierin beschriebenen Proteine und Polypeptide sind vorzugsweise isoliert. Die Begriffe "isoliertes Protein" oder "isoliertes Polypeptid" bedeuten, dass das Protein oder Polypeptid von seiner natürlichen Umgebung getrennt ist. Ein isoliertes Protein oder Polypeptid kann in einem im Wesentlichen aufgereinigten Zustand vorliegen. Der Begriff "im wesentlichen aufgereinigt" bedeutet, dass das Protein oder Polypeptid im Wesentlichen frei von anderen Substanzen vorliegt, mit denen es in der Natur oder *in vivo* vorliegt.

Solche Proteine und Polypeptide dienen beispielsweise der Herstellung von Antikörpern und sind in einem immunologischen oder diagnostischen Assay oder als Therapeutika einsetzbar. Hierin beschriebene Proteine und Polypeptide können aus biologischen Proben wie Gewebe- oder Zellhomogenaten isoliert werden und können auch rekombinant in einer Vielzahl pro- oder eukaryontischer Expressionssysteme exprimiert werden.

"Derivate" eines Proteins oder Polypeptids oder einer Aminosäuresequenz im Sinne dieser Erfindung umfassen Aminosäure-Insertionsvarianten, Aminosäure-Deletionsvarianten und/oder Aminosäure-Substitutionsvarianten.

Aminosäure-Insertionsvarianten umfassen amino- und/oder carboxyterminale Fusionen, sowie Insertionen von einzelnen oder mehreren Aminosäuren in einer bestimmten Aminosäuresequenz. Bei Aminosäure-Sequenzvarianten mit einer Insertion werden ein oder mehrere Aminosäurereste in eine vorbestimmte Stelle in einer Aminosäuresequenz eingebracht, obwohl eine zufällige Insertion mit geeignetem Screening des resultierenden Produkts auch möglich ist. Aminosäure-Deletionsvarianten sind durch das Entfernen von einer oder mehreren Aminosäuren aus der Sequenz charakterisiert. Aminosäure-Substitutionsvarianten zeichnen sich dadurch aus, dass wenigstens ein Rest in der Sequenz entfernt und ein anderer Rest an dessen Stelle eingefügt wird. Vorzugsweise befinden sich die Modifikationen an Positionen in der Aminosäuresequenz, die zwischen homologen Proteinen oder Polypeptiden nicht konserviert sind. Vorzugsweise werden Aminosäuren durch andere mit ähnlichen Eigenschaften, wie Hydrophobizität, Hydrophilizität, Elektronegativität, Volumen der Seitenkette und ähnliches, ersetzt (konservative Substitution). Konservative Substitutionen betreffen beispielsweise den Austausch einer Aminosäure durch eine andere, wobei beide Aminosäuren in derselben nachstehenden Gruppe aufgeführt sind:
1. kleine aliphatische, nicht-polare oder leicht-polare Reste: Ala, Ser, Thr (Pro, Gly)
2. negativ geladene Reste und ihre Amide: Asn, Asp, Glu, Gln
3. positiv geladene Reste: His, Arg, Lys
4. große aliphatische, nicht-polare Reste: Met, Leu, Ile, Val (Cys)
5. große aromatische Reste: Phe, Tyr, Trp.

Drei Reste sind aufgrund ihrer besonderen Rolle für die Proteinarchitektur in Klammern gesetzt. Gly ist der einzige Rest ohne eine Seitenkette und verleiht der Kette somit Flexibilität. Pro besitzt eine ungewöhnliche Geometrie, die die Kette stark einschränkt. Cys kann eine Disulfidbrücke bilden.

Die oben beschriebenen Aminosäure-Varianten können leicht mit Hilfe von bekannten Peptidsynthesetechniken wie z.B. durch "Solid Phase Synthesis" (Merrifield, 1964) und ähnliche Verfahren oder durch rekombinante DNA-Manipulation hergestellt werden. Techniken, um Substitutionsmutationen an vorbestimmten Stellen in DNA einzubringen, die eine bekannte oder teilweise bekannte Sequenz besitzt, sind gut bekannt und umfassen z.B. M13-Mutagenese. Die Manipulation von DNA-Sequenzen zur Herstellung von Proteinen mit Substitutionen, Insertionen oder Deletionen ist z.B. in Sambrook et. al. (1989) ausführlich beschrieben.

"Derivate" von Proteinen oder Polypeptiden, wie hierin verwendet, umfassen auch einzelne oder multiple Substitutionen, Deletionen und/oder Additionen jeglicher Moleküle, die mit dem Enzym assoziiert sind, wie Kohlenhydrate, Lipide und/oder Proteine oder Polypeptide. Ferner erstreckt sich der Begriff "Derivat" auch auf alle funktionellen chemischen Äquivalente der Proteine oder Polypeptide.

Ein Teil oder Fragment eines Tumor-assoziierten Antigens weist eine funktionelle Eigenschaft des Polypeptids auf, aus dem es abgeleitet ist. Solche funktionellen Eigenschaften umfassen die Interaktion mit Antikörpern, die Interaktion mit anderen Polypeptiden oder Proteinen, die selektive Bindung von Nukleinsäuren und eine enzymatische Aktivität. Eine bedeutende Eigenschaft ist die Fähigkeit, einen Komplex mit HLA einzugehen und gegebenenfalls eine Immunreaktion zu erzeugen. Diese Immunreaktion kann auf Stimulation von cytotoxischen oder Helfer-T-Zellen beruhen. Vorzugsweise umfasst ein Teil oder Fragment eines Tumor-assoziierten Antigens eine Sequenz von mindestens 6, insbesondere mindestens 8, mindestens 10, mindestens 12, mindestens 15, mindestens 20, mindestens 30 oder mindestens 50 aufeinanderfolgenden Aminosäuren aus dem Tumor-assoziierten Antigen. Ein Teil oder Fragment eines Tumor-assoziierten Antigens ist vorzugsweise ein Teil des Tumor-assoziierten Antigens, der dem Nichttransmembrananteil, insbesondere dem extrazellulären Anteil des Antigens entspricht oder davon umfasst wird.

Ein Teil oder ein Fragment einer Nukleinsäure, die für ein Tumor-assoziiertes Antigen kodiert, betrifft den Teil der Nukleinsäure, der zumindest für das Tumor-assoziierte Antigen und/oder für einen Teil oder ein Fragment des Tumor-assoziierten Antigens wie vorstehend definiert kodiert. Vorzugsweise ist ein Teil oder Fragment einer Nukleinsäure, die für ein Tumor-assoziiertes Antigen kodiert, derjenige Teil, der dem offenen Leserahmen, insbesondere wie im Sequenzprotokoll angegeben entspricht.

Die Isolierung und Identifizierung von Genen, die für Tumor-assoziierte Antigene kodieren, ermöglicht auch die Diagnose einer Erkrankung, die sich durch die Expression von einem oder mehreren Tumor-assoziierten Antigenen auszeichnet. Diese Verfahren umfassen die Bestimmung einer oder mehrerer Nukleinsäuren, die für ein Tumor-assoziiertes Antigen kodieren, und/oder die Bestimmung der kodierten Tumor-assoziierten Antigene und/oder von davon abgeleiteten Peptiden. Eine Bestimmung der Nukleinsäure kann in herkömmlicher Weise erfolgen, einschließlich durch Polymerase-Kettenreaktion oder Hybridisierung mit einer markierten Sonde. Eine Bestimmung von Tumor-assoziierten Antigenen oder davon abgeleiteten Peptiden kann durch ein Screening von Patienten-Antiseren in Bezug auf eine Erkennung des Antigens und/oder der Peptide erfolgen. Sie kann auch durch ein Screening von T-Zellen des Patienten auf Spezifität für das entsprechende Tumor-assoziierte Antigen erfolgen.

Die hierin beschriebene Strategie ermöglicht auch die Isolierung von Proteinen, die an hier beschriebene Tumor-assoziierte Antigene binden, einschließlich Antikörper und zelluläre Bindepartner der Tumor-assoziierten Antigene.

Hierin werden auch in bestimmten Ausführungsformen "dominant negative" Polypeptide offenbart, die von Tumor-assoziierten Antigenen abgeleitet sind. Ein dominant negatives Polypeptid ist eine inaktive Variante eines Proteins, die durch Interaktion mit der zellulären Maschinerie ein aktives Protein von seiner Interaktion mit der zellulären Maschinerie verdrängt oder mit dem aktiven Protein kompetitiert, wodurch die Wirkung des aktiven Proteins verringert wird. Zum Beispiel kann ein dominant negativer Rezeptor, der einen Liganden bindet, jedoch kein Signal in Reaktion auf die Bindung des Liganden erzeugt, die biologische Wirkung des Liganden verringern. In ähnlicher Weise kann eine dominant negative katalytisch-inaktive Kinase, die normalerweise mit Zielproteinen interagiert, jedoch die Zielproteine nicht phosphoryliert, die Phosphorylierung der Zielproteine in Reaktion auf ein zelluläres Signal verringern. In ähnlicher Weise kann ein dominant negativer Transkriptionsfaktor, der an eine Promotorstelle in der Kontrollregion eines Gens bindet, jedoch die Transkription des Gens nicht erhöht, die Wirkung eines normalen Transkriptionsfaktors durch die Besetzung von Promotorbindestellen ohne eine Erhöhung der Transkription verringern.

Das Ergebnis der Expression eines dominant negativen Polypeptids in einer Zelle ist eine Verringerung der Funktion aktiver Proteine. Der Fachmann kann dominant negative Varianten eines Proteins beispielsweise durch herkömmliche Mutageneseverfahren und Bewerten der dominant negativen Wirkung des Varianten-Polypeptids herstellen.

Umfasst sind auch Stoffe wie Polypeptide, die an Tumor-assoziierte Antigene binden. Solche Bindestoffe können z.B. in Screening-Assays für einen Nachweis von Tumor-assoziierten Antigenen und Komplexen von Tumor-assoziierten Antigenen mit ihren Bindepartnern sowie bei einer Aufreinigung der Tumor-assoziierten Antigene und von Komplexen davon mit ihren Bindepartnern Verwendung finden. Solche Stoffe können auch für eine Hemmung der Aktivität Tumor-assoziierter Antigene beispielsweise durch Bindung an solche Antigene Verwendung finden.

Umfasst sind daher Bindestoffe wie z.B. Antikörper oder Antikörperfragmente, die die Fähigkeit aufweisen, selektiv an Tumor-assoziierte Antigene zu binden. Antikörper umfassen polyklonale und monoklonale Antikörper, die in herkömmlicher Weise hergestellt werden.

Solche Antikörper können Proteine in nativem und/oder denaturiertem Zustand erkennen (Anderson et al., J. Immunol. 143: 1899-1904, 1989; Gardsvoll, J. Immunol. Methods 234: 107-116, 2000; Kayyem et al., Eur. J. Biochem. 208: 1-8, 1992; Spiller et al., J. Immunol. Methods 224: 51-60, 1999).

Antiseren, die spezifische Antikörper enthalten, die an das Zielprotein spezifisch binden, können über verschiedene Standardverfahren hergestellt werden; vgl. beispielsweise "Monoclonal Antibodies: A Practical Approach" von Philip Shepherd, Christopher Dean ISBN 0-19-963722-9, "Antibodies: A Laboratory Manual" von Ed Harlow, David Lane ISBN: 0879693142 und "Using Antibodies: A Laboratory Manual: Portable Protocol NO" von Edward Harlow, David Lane, Ed Harlow ISBN: 0879695447. Dabei ist auch möglich, affine und spezifische Antikörper zu generieren, die komplexe Membranproteine in ihrer nativen Form erkennen (Azorsa et al., J. Immunol. Methods 229: 35-48, 1999; Anderson et al., J. Immunol. 143: 1899-1904, 1989; Gardsvoll, J. Immunol. Methods. 234: 107-116, 2000). Dies ist vor allem für die Herstellung von Antikörpern von Bedeutung, die therapeutisch eingesetzt werden sollen, aber auch für viele diagnostische Anwendungen. Dazu kann sowohl mit dem gesamten Protein, mit extrazellulären Teilsequenzen, wie auch mit Zellen, die das Zielmolekül in physiologisch gefalteter Form exprimieren, immunisiert werden.

Monoklonale Antikörper werden traditionell mit Hilfe der Hybridoma-Technologie hergestellt (Technische Details: siehe "Monoclonal Antibodies: A Practical Approach" von Philip Shepherd, Christopher Dean ISBN 0-19-963722-9; "Antibodies: A Laboratory Manual" von Ed Harlow, David Lane ISBN: 0879693142, "Using Antibodies: A Laboratory Manual: Portable Protocol NO" von Edward Harlow, David Lane, Ed Harlow ISBN: 0879695447).

Es ist bekannt, dass nur ein kleiner Teil eines Antikörpermoleküls, das Paratop, an der Bindung des Antikörpers an sein Epitop beteiligt ist (vgl. Clark, W.R. (1986), The Experimental Foundations of Modern Immunology, Wiley & Sons, Inc., New York; Roitt, I. (1991), Essential Immunology, 7. Auflage, Blackwell Scientific Publications, Oxford). Die pFc'- und Fc-Regionen sind z.B. Effektoren der Komplementkaskade, sind jedoch nicht an der Antigenbindung beteiligt. Ein Antikörper, von dem die pFc'-Region enzymatisch abgespalten wurde oder der ohne die pFc'-Region hergestellt wurde, bezeichnet als F(ab')₂-Fragment, trägt beide Antigenbindestellen eines vollständigen Antikörpers. In ähnlicher Weise trägt ein Antikörper, von dem die Fc-Region enzymatisch abgespalten wurde oder der ohne die Fc-Region hergestellt wurde, bezeichnet als Fab-Fragment, eine Antigenbindestelle eines intakten Antikörpermoleküls. Des Weiteren bestehen Fab-Fragmente aus einer kovalent gebundenen leichten Kette eines Antikörpers und einem Teil der schweren Kette des Antikörpers, bezeichnet als Fd. Die Fd-Fragmente sind die Haupt-Determinanten der Antikörper-Spezifität (ein einzelnes Fd-Fragment kann mit bis zu zehn verschiedenen leichten Ketten assoziiert werden, ohne die Spezifität des Antikörpers zu verändern) und Fd-Fragmente behalten bei einer Isolierung die Fähigkeit, an ein Epitop zu binden.

Innerhalb des Antigen-bindenden Teils eines Antikörpers befinden sich komplementaritätsbestimmende Regionen (CDRs), die direkt mit dem Epitop des Antigens wechselwirken, und Gerüstregionen (FRs), die die Tertiärstruktur des Paratops aufrechterhalten. Sowohl in dem Fd-Fragment der schweren Kette als auch in der leichten Kette von IgG-Immunglobulinen befinden sich vier Gerüstregionen (FR1 bis FR4), die jeweils durch drei komplementaritätsbestimmende Regionen (CDR1 bis CDR3) getrennt sind. Die CDRs und insbesondere die CDR3-Regionen und noch mehr die CDR3-Region der schweren Kette sind größtenteils für die Antikörper-Spezifität verantwortlich.

Man weiß, dass die Nicht-CDR-Regionen eines Säuger-Antikörpers durch ähnliche Regionen von Antikörpern mit der gleichen oder einer anderen Spezifität ersetzt werden können, wobei die Spezifität für das Epitop des ursprünglichen Antikörpers erhalten bleibt. Dies ermöglichte die Entwicklung sogenannter "humanisierter" Antikörper, bei denen nicht-menschliche CDRs kovalent mit menschlichen FR- und/oder Fc/pFc'-Regionen für die Herstellung eines funktionellen Antikörpers verbunden sind.

Dies nutzt die sogenannte "SLAM"-Technologie. Hierbei werden B-Zellen aus Vollblut isoliert und die Zellen monoklonalisiert. Anschließend wird der Überstand der vereinzelten B-Zelle auf ihre Antikörperspezifität hin analysiert. Im Gegensatz zur Hybridomatechnologie wird anschließend die variable Region des Antikörpergens durch eine Einzelzell-PCR amplifiziert und in einen geeigneten Vektor kloniert. Auf diese Art und Weise wird die Gewinnung von monoklonalen Antikörpern beschleunigt (de Wildt et al. J. Immunol. Methods 207:61-67, 1997).

Als anderes Beispiel beschreibt die WO 92/04381 die Herstellung und Verwendung von humanisierten RSV-Antikörpern aus Maus, bei denen mindestens ein Teil der FR-Regionen aus Maus durch FR-Regionen eines menschlichen Ursprungs ersetzt wurden. Solche Antikörper, einschließlich Fragmente intakter Antikörper mit einer Antigen-Bindefähigkeit werden oft als "chimäre" Antikörper bezeichnet.

Hierin werden auch F(ab')₂-, Fab-, Fv- und Fd-Fragmente von Antikörpern, chimäre Antikörper, bei denen die Fc- und/oder FR- und/oder CDR1- und/oder CDR2- und/oder leichte Kette-CDR3-Regionen durch homologe menschliche oder nicht-menschliche Sequenzen ersetzt wurden, chimäre F(ab')₂-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, chimäre Fab-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, und chimäre Fd-Fragment-Antikörper, bei denen die FR- und/oder CDR1- und/oder CDR2-Regionen durch homologe menschliche oder nicht-menschliche Sequenzen ersetzt wurden, offenbart. Auch umfasst sind sogenannte einzelkettige Antikörper.

Vorzugsweise ist ein erfindungsgemäß verwendeter Antikörper gegen eine der Sequenzen gemäß SEQ ID NOs: 6, 282 oder 283des Sequenzprotokolls, einen Teil oder ein Derivat davon, insbesondere eine Sequenz gemäß SEQ ID NO: 282 oder 283 des Sequenzprotokolls gerichtet und/oder kann durch Immunisierung mit diesen Peptiden erhalten werden.

Offenbart werden auch Polypeptide, die spezifisch an Tumor-assoziierte Antigene binden. Beispielsweise können solche Polypeptid-Bindestoffe durch degenerierte Peptid-Bibliotheken bereitgestellt werden, die einfach in Lösung in einer immobilisierten Form oder als Phagen-Display-Bibliotheken hergestellt werden können. Kombinatorische Bibliotheken aus Peptiden mit einer oder mehreren Aminosäuren können ebenfalls hergestellt werden. Ferner können Bibliotheken aus Peptoiden und nicht peptidischen synthetischen Resten hergestellt werden.

Phagen-Display kann besonders wirksam bei der Identifizierung hierin beschriebener Bindepeptide sein. Dabei wird beispielsweise eine Phagen-Bibliothek (durch Verwendung beispielsweise des m13-, fd- oder lambda-Phagen) hergestellt, die Inserts einer Länge von 4 bis etwa 80 Aminosäureresten präsentiert. Es werden sodann Phagen ausgewählt, die Inserts tragen, die an das Tumor-assoziierte Antigen binden. Dieser Prozess kann über mehrere Zyklen einer Rückselektion von Phagen wiederholt werden, die an das Tumor-assoziierte Antigen binden. Wiederholte Runden führen zu einer Anreicherung von Phagen, die bestimmte Sequenzen tragen. Es kann eine Analyse von DNA-Sequenzen erfolgen, um die Sequenzen der exprimierten Polypeptide zu identifizieren. Der kleinste lineare Anteil der Sequenz, der an das Tumor-assoziierte Antigen bindet, kann bestimmt werden. Das "twohybrid-System" aus Hefe kann auch für die Identifizierung von Polypeptiden eingesetzt werden, die an ein Tumor-assoziiertes Antigen binden. Hierin beschriebene Tumor-assoziierte Antigene oder Fragmente davon können für ein Screening von Peptid-Bibliotheken, einschließlich Phagen-Display-Bibliotheken, eingesetzt werden, um Peptid-Bindepartner der Tumor-assoziierten Antigene zu identifizieren und selektieren. Solche Moleküle können beispielsweise für Screening-Assays, Aufreinigungsprotokolle, für eine Interferenz mit der Funktion des Tumor-assoziierten Antigens und für andere Zwecke, die dem Fachmann bekannt sind, verwendet werden.

Die vorstehend beschriebenen Antikörper und andere Bindemoleküle können beispielsweise für die Identifizierung von Gewebe verwendet werden, das ein Tumor-assoziiertes Antigen exprimiert. Antikörper können auch an spezifische diagnostische Stoffe für eine Darstellung von Zellen und Geweben gekoppelt werden, die Tumor-assoziierte Antigene exprimieren. Sie können ferner an therapeutisch nützliche Stoffe gekoppelt werden. Diagnostische Stoffe umfassen in nicht begrenzender Weise Bariumsulfat, Iocetaminsäure, Iopansäure, Calcium-Ipodat, Natrium-Diatrizoat, Meglumin-Diatrizoat, Metrizamid, Natrium-Tyropanoat und Radiodiagnostika, einschließlich Positronen-Emitter wie Fluor-18 und Kohlenstoff-11, gamma-Emitter wie Iod-123, Technetium-99m, Iod-131 und Indium-111, Nuklide für magnetische Kernresonanz wie Fluor und Gadolinium. Der Begriff "therapeutisch nützlicher Stoff", wie hierin verwendet, meint jedes therapeutische Molekül, das wunschgemäß selektiv zu einer Zelle geführt wird, die ein oder mehrere Tumor-assoziierte Antigene exprimiert, einschließlich Antikrebsmittel, mit radioaktivem Iod versehene Verbindungen, Toxine, cytostatische oder cytolytische Arzneistoffe, usw. Antikrebsmittel umfassen beispielsweise Aminoglutethimid, Azathioprin, Bleomycinsulfat, Busulfan, Carmustin, Chlorambucil, Cisplatin, Cyclophosphamid, Cyclosporin, Cytarabin, Dacarbazin, Dactinomycin, Daunorubin, Doxorubicin, Taxol, Etoposid, Fluoruracil, Interferon-α, Lomustin, Mercaptopurin, Methotrexat, Mitotan, Procarbazin-HCl, Thioguanin, Vinblastinsulfat und Vincristinsulfat. Weitere Antikrebsmittel sind beispielsweise in Goodman und Gilman, "The Pharmacological Basis of Therapeutics", 8. Auflage, 1990, McGraw-Hill, Inc., insbesondere Kapitel 52 (Antineoplastic Agents (Paul Calabresi und Bruce A. Chabner)) beschrieben. Toxine können Proteine wie Pokeweed-antivirales Protein, Choleratoxin, Pertussistoxin, Ricin, Gelonin, Abrin, Diphtherie-Exotoxin oder *Pseudomonas-Exotoxin* sein. Toxinreste können auch Hochenergie-emittierende Radionuklide wie Kobalt-60 sein.

Der Begriff "Patient", wie hierin verwendet, bedeutet Mensch, nicht menschlicher Primat oder ein anderes Tier, insbesondere Säugetier wie Kuh, Pferd, Schwein, Schaf, Ziege, Hund, Katze oder Nagetier wie Maus und Ratte. In einer besonders bevorzugten Ausführungsform ist der Patient ein Mensch.

Der Begriff "Erkrankung", wie hierin verwendet, betrifft jeden pathologischen Zustand, bei dem Tumor-assoziierte Antigene exprimiert oder abnormal exprimiert werden. "Abnormale Expression", wie hierin verwendet, bedeutet, dass die Expression gegenüber dem Zustand bei einem gesunden Individuum verändert, vorzugsweise erhöht ist. Eine Erhöhung der Expression betrifft eine Erhöhung um mindestens 10%, insbesondere mindestens 20%, mindestens 50% oder mindestens 100%. In einer Ausführungsform wird das Tumor- assoziierte Antigen nur in Gewebe eines erkrankten Individuums exprimiert, während die Expression bei einem gesunden Individuum reprimiert ist. Ein Beispiel einer solchen Erkrankung ist Krebs, wobei der Begriff "Krebs", wie hierin verwendet, Leukämien, Seminome, Melanome, Teratome, Gliome, Darm-, Colon-, Rektal-, Kolorektal-, Magen-, Gastrointestinal-, Speiseröhren-, Hals, Nasen, Ohren (HNO)-, Nieren-, Nebennieren-, Schilddrüsen-, Lymphknoten-, Brust-, Prostata-, Gebärmutter-, Ovarial-, Endometrial-, Leber, Pankreas-, Haut-, Gehirn- und Lungenkrebs und deren Metastasen umfasst.

Eine biologische Probe, wie hierin verwendet, kann eine Gewebe- und/oder zelluläre Probe sein und kann für eine Verwendung in den verschiedenen, hier beschriebenen Verfahren in herkömmlicher Weise gewonnen werden, wie durch Gewebebiopsie, einschließlich Stanzbiopsie, und Entnahme von Blut, Bronchialaspirat, Urin, Fäces oder anderen Körperflüssigkeiten.

Der Begriff "immunreaktive Zelle", wie hierin verwendet, bedeutet eine Zelle, die in eine Immunzelle (wie B-Zelle, Helfer-T-Zelle oder cytolytische T-Zelle) bei geeigneter Stimulierung reifen kann. Immunreaktive Zellen umfassen CD34⁺ hämatopoietische Stammzellen, unreife und reife T-Zellen sowie unreife und reife B-Zellen. Falls die Herstellung cytolytischer oder Helfer-T-Zellen, die ein Tumor-assoziiertes Antigen erkennen, gewünscht ist, wird die immunreaktive Zelle mit einer Zelle, die ein Tumor-assoziiertes Antigen exprimiert, unter Bedingungen in Kontakt gebracht, die eine Produktion, Differenzierung und/oder Selektion von cytolytischen sowie Helfer-T-Zellen begünstigen. Die Differenzierung von T-Zell-Vorläufern in eine cytolytische T-Zelle bei einer Exposition gegenüber einem Antigen ist ähnlich zur klonalen Selektion des Immunsystems.

Manche therapeutische Verfahren beruhen auf einer Reaktion des Immunsystems eines Patienten, die zu einer Lyse Antigen-präsentierender Zellen führt, wie Krebszellen, die ein oder mehrere Tumor-assoziierte Antigene präsentieren. Dabei werden beispielsweise autologe cytotoxische T-Lymphozyten, die für einen Komplex aus einem Tumor-assoziierten Antigen und einem MHC-Molekül spezifisch sind, an einen Patienten mit einer Zellabnormalie verabreicht. Die Produktion solcher cytotoxischer T-Lymphozyten *in vitro* ist bekannt. Ein Beispiel für ein Verfahren zur Differenzierung von T-Zellen findet sich in der WO-A-96/33265. Im Allgemeinen wird eine Probe mit Zellen wie Blutzellen aus dem Patienten entnommen und die Zellen werden mit einer Zelle in Kontakt gebracht, die den Komplex präsentiert und eine Vermehrung von cytotoxischen T-Lymphozyten auslösen kann (z.B. dendritische Zellen). Die Zielzelle kann eine transfizierte Zelle wie eine COS-Zelle sein. Diese transfizierten Zellen präsentieren den gewünschten Komplex auf ihrer Oberfläche und stimulieren bei einer Kontaktierung mit cytotoxischen T-Lymphozyten deren Vermehrung. Die klonal expandierten autologen cytotoxischen T-Lymphozyten werden sodann an den Patienten verabreicht.

Bei einem anderen Verfahren zur Selektion Antigen-spezifischer cytotoxischer T- Lymphozyten werden fluorogene Tetramere von MHC-Klasse I-Molekül/Peptid-Komplexen für einen Nachweis spezifischer Klone von cytotoxischen T-Lymphozyten verwendet (Altman et al., Science 274:94-96, 1996; Dunbar et al., Curr. Biol. 8:413-416, 1998). Lösliche MHC-Klasse I-Moleküle werden *in vitro* in Gegenwart von β₂-Mikroglobulin und eines Peptid-Antigens, das an das Klasse I-Molekül bindet, gefaltet. Nach Aufreinigung der MHC/Peptid-Komplexe werden diese mit Biotin markiert. Tetramere werden durch Mischen der biotinylierten Peptid-MHC-Komplexe mit markiertem Avidin (z.B. Phycoerythrin) bei einem molaren Verhältnis von 4:1 gebildet. Tetramere werden sodann mit cytotoxischen T-Lymphozyten wie peripherem Blut oder Lymphknoten in Kontakt gebracht. Die Tetramere binden an cytotoxische T-Lymphozyten, die den Peptid-Antigen/MHC-Klasse I-Komplex erkennen. Zellen, die an die Tetramere gebunden werden, können durch Fluoreszenzgesteuerte Zellsortierung für eine Isolierung reaktiver cytotoxischer T-Lymphozyten sortiert werden. Die isolierten cytotoxischen T-Lymphozyten können sodann *in vitro* vermehrt werden.

Bei einem therapeutischen Verfahren, das als adoptiver Transfer bezeichnet wird (Greenberg, J. Immunol. 136(5):1917, 1986; Riddel et al., Science 257:238, 1992; Lynch et al., Eur. J. Immunol. 21:1403-1410, 1991; Kast et al., Cell 59:603-614, 1989), werden Zellen, die den gewünschten Komplex präsentieren (z.B. dendritische Zellen), mit cytotoxischen T-Lymphozyten des zu behandelnden Patienten kombiniert, was zu einer Vermehrung spezifischer cytotoxischer T-Lymphozyten führt. Die vermehrten cytotoxischen T-Lymphozyten werden sodann an einen Patienten mit einer zellulären Abnormalie verabreicht, die sich durch bestimmte abnormale Zellen auszeichnet, die den spezifischen Komplex präsentieren. Die cytotoxischen T-Lymphozyten lysieren sodann die abnormalen Zellen, wodurch eine gewünschte therapeutische Wirkung erreicht wird.

Oft lassen sich aus dem T-Zell-Repertoire eines Patienten lediglich niedrig-affine T-Zellen gegen einen solchen spezifischen Komplex vermehren, da die hochaffinen durch Toleranzentwicklung ausgelöscht worden sind. Eine Alternative kann hier ein Transfer des T-Zell-Rezeptors selbst sein. Hierfür werden ebenfalls Zellen, die den gewünschten Komplex präsentieren (z.B. dendritische Zellen), mit cytotoxischen T-Lymphozyten von Gesunden kombiniert. Dies führt zu einer Vermehrung hochaffiner spezifischer cytotoxischer T-Lymphozyten, wenn die T-Lymphozyten aus einem Spenderorganismus kommen, der mit dem spezifischen Komplex bisher keinen Kontakt hatte. Der hochaffine T-Zell-Rezeptor aus diesen vermehrten spezifischen T-Lymphozyten wird kloniert und kann durch Gentransfer z.B. mit retroviralen Vektoren beliebig in T-Zellen von anderen Patienten transduziert werden. Adoptiver Transfer erfolgt dann mit diesen genetisch veränderten T-Lymphozyten (Stanislawski et al., Nat. Immunol. 2:962-70, 2001; Kessels et al., Nat. Immunol. 2:957-61, 2001).

Die vorstehenden therapeutischen Aspekte gehen davon aus, dass zumindest manche der abnormalen Zellen des Patienten einen Komplex aus einem Tumor-assoziierten Antigen und einem HLA-Molekül präsentieren. Eine Identifizierung solcher Zellen kann in an sich bekannter Weise erfolgen. Sobald Zellen, die den Komplex präsentieren, identifiziert wurden, können sie mit einer Probe aus dem Patienten, die cytotoxische T-Lymphozyten enthält, kombiniert werden. Falls die Zellen, die den Komplex präsentieren, durch die cytotoxischen T-Lymphozyten lysiert werden, kann angenommen werden, dass ein Tumor-assoziiertes Antigen präsentiert wird.

Der adoptive Transfer ist nicht die einzige Therapieform, die hierbei anwendbar ist. Cytotoxische T-Lymphozyten können auch *in vivo* in an sich bekannter Weise erzeugt werden. Bei einem Verfahren werden nicht proliferative Zellen verwendet, die den Komplex exprimieren. Die Zellen, die dabei verwendet werden, werden diejenigen sein, die normalerweise den Komplex exprimieren, wie bestrahlte Tumorzellen oder Zellen, die mit einem oder beiden Genen transfiziert wurden, die für eine Präsentation des Komplexes notwendig sind (d.h. das Antigen-Peptid und das präsentierende HLA-Molekül). Verschiedene Zelltypen können eingesetzt werden. Des Weiteren können Vektoren verwendet werden, die eines oder beide der interessierenden Gene tragen. Virale oder bakterielle Vektoren sind besonders bevorzugt. Zum Beispiel können Nukleinsäuren, die für ein Tumor-assoziiertes Antigen oder einen Teil davon kodieren, funktionell mit Promotor- und Enhancersequenzen verknüpft werden, die eine Expression des Tumor-assoziierten Antigens oder eines Fragments davon in bestimmten Geweben oder Zelltypen steuern. Die Nukleinsäure kann in einen Expressionsvektor eingebaut werden. Expressionsvektoren können nicht-modifizierte extrachromosomale Nukleinsäuren, Plasmide oder virale Genome sein, in die eine Insertion exogener Nukleinsäuren möglich ist. Nukleinsäuren, die für ein Tumor-assoziiertes Antigen kodieren, können auch in ein retrovirales Genom inseriert werden, wodurch die Integration der Nukleinsäure in das Genom des Zielgewebes oder der Zielzelle ermöglicht wird. Bei diesen Systemen trägt ein Mikroorganismus wie Vacciniavirus, Poxvirus, Herpes simplex-Virus, Retrovirus oder Adenovirus das interessierende Gen und "infiziert" de facto Wirtszellen. Eine weitere bevorzugte Form ist die Einbringung des Tumor-assoziierten Antigens in Form von rekombinanter RNA. Diese kann z.B. durch liposomalen Transfer oder durch Elektroporation in Zellen eingebracht werden. Die resultierenden Zellen präsentieren den interessierenden Komplex und werden von autologen cytotoxischen T-Lymphozyten erkannt, die sich sodann vermehren.

Eine ähnliche Wirkung kann durch Kombination des Tumor-assoziierten Antigens oder eines Fragments davon mit einem Adjuvans erreicht werden, um einen Einbau in Antigen-präsentierende Zellen *in vivo* zu ermöglichen. Das Tumor-assoziierte Antigen oder ein Fragment davon können als Protein, als DNA (z.B. innerhalb eines Vektors) oder als RNA repräsentiert sein. Das Tumor-assoziierte Antigen wird prozessiert, um einen Peptidpartner für das HLA-Molekül zu ergeben, während ein Fragment davon präsentiert werden kann, ohne dass eine weitere Prozessierung erforderlich ist. Letzteres ist insbesondere der Fall, wenn diese an HLA-Moleküle binden können. Verabreichungsformen, bei denen das Gesamt-Antigen *in vivo* von einer dendritischen Zelle prozessiert wird, sind bevorzugt, da hier auch Helfer-T-Zell-Antworten entstehen können. Eine effektive Immunantwort benötigt diese (Ossendorp et al., Immunol. Lett. 74:75-9, 2000; Ossendorp et al., J. Exp. Med. 187:693-702, 1998). Im Allgemeinen kann eine wirksame Menge des Tumor-assoziierten Antigens an einen Patienten z.B. durch eine intradermale Injektion verabreicht werden. Die Injektion kann aber auch intranodal in einen Lymphknoten erfolgen (Maloy et al., Proc. Natl. Acad. Sci. USA 98:3299-303, 2001). Sie kann auch in Kombination mit Reagenzien erfolgen, die eine Aufnahme in dendritische Zellen erleichtern. Bevorzugte Tumor-assoziierte Antigene umfassen diejenigen, die mit allogenen Krebs-Antiseren oder mit T-Zellen vieler Krebs-Patienten reagieren. Von besonderem Interesse sind aber auch solche, gegen die keine spontanen Immunantworten vorbestehen. Gegen diese können nachweislich Immunantworten induziert werden, die Tumoren lysieren können (Keogh et al., J. Immunol. 167:787-96, 2001; Appella et al., Biomed. Pept. Proteins Nucleic Acids 1:177-84, 1995; Wentworth et al., Mol. Immunol. 32:603-12, 1995).

Die offenbarten pharmazeutischen Zusammensetzungen können auch als Vakzinen für die Immunisierung eingesetzt werden. Die Begriffe "Immunisierung" oder "Vakzinierung", wie hierin verwendet, bedeuten eine Erhöhung oder Aktivierung einer Immunreaktion gegenüber einem Antigen. Tiermodelle können für ein Testen einer immunisierenden Wirkung gegenüber Krebs durch Verwendung eines Tumor-assoziierten Antigens oder einer dafür kodierenden Nukleinsäure eingesetzt werden. Zum Beispiel können menschliche Krebszellen in eine Maus für die Schaffung eines Tumors eingebracht werden und eine oder mehrere Nukleinsäuren, die für Tumor-assoziierte Antigene kodieren, können verabreicht werden. Die Wirkung auf die Krebszellen (beispielsweise Verringerung der Tumorgröße) kann als Maß für die Wirksamkeit einer Immunisierung durch die Nukleinsäure gemessen werden.

Als Teil der Zusammensetzung für eine Immunisierung werden eines oder mehrere Tumor- assoziierte Antigene oder stimulierende Fragmente davon mit einem oder mehreren Adjuvanzien für eine Induktion einer Immunreaktion oder eine Erhöhung einer Immunreaktion verabreicht. Ein Adjuvans ist eine Substanz, die in das Antigen eingebaut oder gemeinsam mit diesem verabreicht wird und die Immunreaktion verstärkt. Adjuvanzien können die Immunreaktion durch Bereitstellen eines Antigen-Reservoirs (extrazellulär oder in Makrophagen), Aktivierung von Makrophagen und/oder Stimulierung bestimmter Lymphozyten verstärken. Adjuvanzien sind bekannt und umfassen in nicht begrenzender Weise Monophosphoryl-Lipid-A (MPL, SmithKline Beecham), Saponine wie QS21 (SmithKline Beecham), DQS21 (SmithKline Beecham; WO 96/33739), QS7, QS17, QS18 und QS-L1 (So et al., Mol. Cells 7:178-186, 1997), unvollständiges Freundsches Adjuvans, vollständiges Feundsches Adjuvans, Vitamin E, Montanid, Alaun, CpG-Nukleotide (vgl. Krieg et al., Nature 374:546-9, 1995) und verschiedene Wasser-in-Öl-Emulsionen, die aus biologisch abbaubaren Ölen wie Squalen und/oder Tocopherol hergestellt werden. Vorzugsweise werden die Peptide in einer Mischung mit DQS21/MPL verabreicht. Das Verhältnis von DQS21 zu MPL beträgt typischerweise etwa 1:10 bis 10:1, vorzugsweise etwa 1:5 bis 5:1 und insbesondere etwa 1:1. Für eine Verabreichung an den Menschen sind DQS21 und MPL typischerweise in einer Vakzine-Formulierung in einem Bereich von etwa 1 µg bis etwa 100 µg vorhanden.

Andere Stoffe, die eine Immunreaktion des Patienten stimulieren, können auch verabreicht werden. Zum Beispiel sind Zytokine bei einer Vakzinierung aufgrund ihrer regulatorischen Eigenschaften auf Lymphozyten verwendbar. Solche Zytokine umfassen z.B. Interleukin-12 (IL-12), von dem gezeigt wurde, dass es die schützenden Wirkungen von Vakzinen verstärkt (vgl. Science 268:1432-1434, 1995), GM-CSF und IL-18.

Es gibt eine Reihe von Verbindungen, die eine Immunreaktion verstärken und die daher bei einer Vakzinierung eingesetzt werden können. Diese umfassen co-stimulierende Moleküle, die in Form von Proteinen oder Nukleinsäuren bereitgestellt werden. Solche co-stimulierenden Moleküle sind beispielsweise B7-1 und B7-2 (CD80 bzw. CD86), die auf dendritischen Zellen (DC) exprimiert werden und mit dem auf den T-Zellen exprimierten CD28-Molekül interagieren. Diese Interaktion stellt eine Co-Stimulierung (Signal 2) für eine Antigen/MHC/TCR-stimulierte (Signal 1) T-Zelle bereit, wodurch die Vermehrung der T-Zelle und die Effektorfunktion verstärkt wird. B7 interagiert auch mit CTLA4 (CD 152) auf T-Zellen und Untersuchungen, die CTLA4- und B7-Liganden einbeziehen, zeigen, dass die B7-CTLA4-Interaktion eine Antitumor-Immunität und CTL-Vermehrung verstärken kann (Zheng, P. et al., Proc. Natl. Acad. Sci. USA 95(11):6284-6289 (1998)).

B7 wird typischerweise nicht auf Tumorzellen exprimiert, so dass diese keine wirksamen Antigen-präsentierenden Zellen (APCs) für T-Zellen sind. Eine Induktion der B7-Expression würde ermöglichen, dass Tumorzellen wirksamer eine Vermehrung von cytotoxischen T-Lymphozyten und eine Effektorfunktion stimulieren. Eine Co-Stimulierung durch eine Kombination von B7/IL-6/IL-12 zeigte eine Induktion des IFN-gamma- und Th1-Zytokin-Profils in einer T-Zell-Population, was zu einer weiter verstärkten T-Zell-Aktivität führt (Gajewski et al., J. Immunol. 154:5637-5648 (1995)).

Eine vollständige Aktivierung von cytotoxischen T-Lymphozyten und eine vollständige Effektorfunktion erfordert eine Mitwirkung von T-Helferzellen durch die Interaktion zwischen dem CD40-Liganden auf den T-Helferzellen und dem CD40-Molekül, das von dendritischen Zellen exprimiert wird (Ridge et al., Nature 393:474 (1998), Bennett et al., Nature 393:478 (1998), Schönberger et al., Nature 393:480 (1998)). Der Mechanismus dieses co-stimulierenden Signals betrifft wahrscheinlich die Steigerung der B7- und assoziierten IL-6/IL-12-Produktion durch die dendritischen Zellen (Antigen-präsentierenden Zellen). Die CD40-CD40L-Interaktion komplementiert so die Interaktionen des Signals 1 (Antigen/MHC-TCR) und des Signals 2 (B7-CD28).

Die Verwendung von anti-CD40-Antikörpern für eine Stimulierung von dendritischen Zellen würde erwartungsgemäß direkt eine Reaktion gegenüber Tumor-Antigenen verstärken, die normalerweise außerhalb des Bereichs einer entzündlichen Reaktion liegen oder von nichtprofessionellen Antigen-präsentierenden Zellen (Tumorzellen) präsentiert werden. In diesen Situationen werden T-Helfer- und B7-co-stimulierende Signale nicht bereitgestellt. Dieser Mechanismus könnte im Zusammenhang mit Therapien verwendet werden, die auf Antigengepulsten dendritischen Zellen basieren, oder in Situationen, bei denen T-Helfer-Epitope nicht in bekannten TRA-Vorläufern definiert wurden.

Hierin wird auch eine Verabreichung von Nukleinsäuren, Polypeptiden oder Peptiden offenbart. Eine Verabreichung von Polypeptiden und Peptiden kann in an sich bekannter Weise erfolgen. In einer Ausführungsform erfolgt die Verabreichung von Nukleinsäuren durch ex vivo-Verfahren, d.h. durch Entfernung von Zellen aus einem Patienten, genetische Veränderung der Zellen, um ein Tumor-assoziiertes Antigen einzubauen, und Wiedereinbringung der veränderten Zellen in den Patienten. Dies umfasst im Allgemeinen das Einbringen einer funktionellen Kopie eines Gens in die Zellen eines Patienten *in vitro* und die Rückführung der genetisch veränderten Zellen in den Patienten. Die funktionelle Kopie des Gens steht unter funktioneller Kontrolle von regulatorischen Elementen, die eine Expression des Gens in den genetisch veränderten Zellen erlauben. Transfektions- und Transduktionsverfahren sind dem Fachmann bekannt. Offenbart ist auch eine Verabreichung von Nukleinsäuren *in vivo* durch die Verwendung von Vektoren wie Viren und zielgesteuerten Liposomen.

In einer bevorzugten Ausführungsform ist ein viraler Vektor für die Verabreichung einer Nukleinsäure, die für ein Tumor-assoziiertes Antigen kodiert, aus der Gruppe ausgewählt bestehend aus Adenoviren, Adeno-assoziierten Viren, Poxviren, einschließlich Vacciniavirus und attenuierten Poxviren, Semliki-Forest-Virus, Retroviren, Sindbis-Virus und Ty-Virus- ähnlichen Partikeln. Besonders bevorzugt sind Adenoviren und Retroviren. Die Retroviren sind üblicherweise replikationsdefizient (d.h. sie sind unfähig, infektiöse Partikel zu erzeugen).

Verschiedene Verfahren können eingesetzt werden, um Nukleinsäuren in Zellen *in vitro* oder *in vivo* einzubringen. Solche Verfahren umfassen die Transfektion von Nukleinsäure-Kalziumphosphat-Präzipitaten, die Transfektion von Nukleinsäuren, die mit DEAE assoziiert sind, die Transfektion oder Infektion mit den vorstehenden Viren, die die interessierenden Nukleinsäuren tragen, die Liposomen-vermittelte Transfektion und ähnliches. In bestimmten Ausführungsformen ist eine Steuerung der Nukleinsäure an bestimmte Zellen bevorzugt. In solchen Ausführungsformen kann ein Träger, der für die Verabreichung einer Nukleinsäure an eine Zelle (z.B. ein Retrovirus oder ein Liposom) eingesetzt wird, ein gebundenes Zielsteuerungsmolekül aufweisen. Zum Beispiel kann ein Molekül wie ein Antikörper, der für ein Oberflächenmembran-Protein auf der Zielzelle spezifisch ist, oder ein Ligand für einen Rezeptor auf der Zielzelle in den Nukleinsäureträger eingebaut oder daran gebunden werden. Bevorzugte Antikörper umfassen Antikörper, die selektiv ein Tumor-assoziiertes Antigen binden. Falls eine Verabreichung einer Nukleinsäure durch Liposomen erwünscht ist, können Proteine, die an ein Oberflächenmembran-Protein binden, das mit der Endozytose assoziiert ist, in die Liposomenformulierung eingebaut werden, um eine Zielsteuerung und/oder Aufnahme zu ermöglichen. Solche Proteine umfassen Kapsid-Proteine oder Fragmente davon, die für einen bestimmten Zelltyp spezifisch sind, Antikörper gegen Proteine, die internalisiert werden, Proteine, die eine intrazelluläre Stelle ansteuern, und ähnliches.

Die offenbarten therapeutischen Zusammensetzungen können in pharmazeutisch verträglichen Zubereitungen verabreicht werden. Solche Zubereitungen können gewöhnlich pharmazeutisch verträgliche Konzentrationen von Salzen, Pufferstoffen, Konservierungsstoffen, Trägern, ergänzenden immunitätssteigernden Stoffen wie Adjuvanzien (z.B. CpG-Nukleotide) und Zytokinen und gegebenenfalls andere therapeutische Wirkstoffe enthalten.

Die offenbarten therapeutischen Wirkstoffe können auf jedem herkömmlichen Weg verabreicht werden, einschließlich durch Injektion oder durch Infusion. Die Verabreichung kann beispielsweise oral, intravenös, intraperitoneal, intramuskulär, subkutan oder transdermal erfolgen. Eine therapeutische Verabreichung von Antikörpern erfolgt vorzugsweise durch ein Lungenaerosol. Die Verabreichung von Antisense-Nukleinsäuren erfolgt vorzugsweise durch langsame intravenöse Verabreichung.

Die offenbarten Zusammensetzungen werden in wirksamen Mengen verabreicht. Eine "wirksame Menge" betrifft die Menge, die alleine oder zusammen mit weiteren Dosen eine

gewünschte Reaktion oder eine gewünschte Wirkung erzielt. Im Fall einer Behandlung einer bestimmten Erkrankung oder eines bestimmten Zustands, der sich durch die Expression eines oder mehrerer Tumor-assoziierter Antigene auszeichnet, betrifft die gewünschte Reaktion die Hemmung des Krankheitsverlaufs. Dies umfasst die Verlangsamung des Fortschreitens der Erkrankung und insbesondere eine Unterbrechung des Fortschreitens der Erkrankung. Die gewünschte Reaktion bei einer Behandlung einer Erkrankung oder eines Zustands kann auch die Verzögerung des Ausbruchs oder eine Verhinderung des Ausbruchs der Erkrankung oder des Zustands sein.

Eine wirksame Menge einer offenbarten Zusammensetzung wird von dem zu behandelnden Zustand, der Schwere der Krankheit, den individuellen Parametern des Patienten, einschließlich Alter, physiologischer Zustand, Größe und Gewicht, der Dauer der Behandlung, der Art einer begleitenden Therapie (falls vorhanden), dem spezifischen Verabreichungsweg und ähnlichen Faktoren abhängen.

Die offenbarten pharmazeutischen Zusammensetzungen sind vorzugsweise steril und enthalten eine wirksame Menge der therapeutisch wirksamen Substanz für die Erzeugung der gewünschten Reaktion oder der gewünschten Wirkung.

Die Dosen der offenbarten Zusammensetzungen, die verabreicht werden, können von verschiedenen Parametern wie der Verabreichungsart, dem Zustand des Patienten, dem gewünschten Verabreichungszeitraum, usw. abhängen. Für den Fall, dass eine Reaktion bei einem Patienten bei einer anfänglichen Dosis unzureichend ist, können höhere Dosen (oder effektiv höhere Dosen, die durch einen anderen, stärker lokalisierten Verabreichungsweg erzielt werden) eingesetzt werden.

Im Allgemeinen werden für eine Behandlung oder für eine Erzeugung oder Erhöhung einer Immunreaktion Dosen des Tumor-assoziierten Antigens von 1 ng bis 1 mg, vorzugsweise von 10 ng bis 100 µg formuliert und verabreicht. Falls die Verabreichung von Nukleinsäuren (DNA sowie RNA), die für Tumor-assoziierte Antigene kodieren, erwünscht ist, werden Dosen von 1 ng bis 0,1 mg formuliert und verabreicht.

Die offenbarten pharmazeutischen Zusammensetzungen werden im Allgemeinen in pharmazeutisch verträglichen Mengen und in pharmazeutisch verträglichen Zusammensetzungen verabreicht. Der Begriff "pharmazeutisch verträglich" betrifft ein nichttoxisches Material, das nicht mit der Wirkung des aktiven Bestandteils der pharmazeutischen Zusammensetzung wechselwirkt. Solche Zubereitungen können gewöhnlich Salze, Pufferstoffe, Konservierungsstoffe, Träger und gegebenenfalls andere therapeutische Wirkstoffe enthalten. Bei einer Verwendung in der Medizin sollten die Salze pharmazeutisch verträglich sein. Nicht-pharmazeutisch verträgliche Salze können jedoch für die Herstellung pharmazeutisch verträglicher Salze davon verwendet werden und sind umfasst. Solche pharmakologisch und pharmazeutisch verträglichen Salze umfassen in nicht begrenzender Weise diejenigen, die aus den nachstehenden Säuren hergestellt werden: Chlorwasserstoff-, Bromwasserstoff-, Schwefel-, Salpeter-, Phosphor-, Malein-, Essig-, Salicyl-, Citronen-, Ameisen-, Malon-, Bernsteinsäure und ähnliches. Pharmazeutisch verträgliche Salze können auch als Alkalimetall- oder Erdalkalimetallsalze wie Natrium-, Kalium- oder Calciumsalze hergestellt werden.

Eine offenbarte pharmazeutische Zusammensetzung kann einen pharmazeutisch verträglichen Träger umfassen. Der Begriff "pharmazeutisch verträglicher Träger" betrifft einen oder mehrere kompatible feste oder flüssige Füllstoffe, Verdünnungsmittel oder Kapselsubstanzen, die für eine Verabreichung an einen Menschen geeignet sind. Der Begriff "Träger" betrifft einen organischen oder anorganischen Bestandteil, natürlicher oder synthetischer Natur, in dem der aktive Bestandteil kombiniert wird, um eine Anwendung zu erleichtern. Die Bestandteile der offenbarten pharmazeutischen Zusammensetzung sind gewöhnlich derart, dass keine Interaktion auftritt, die die gewünschte pharmazeutische Wirksamkeit wesentlich beeinträchtigt.

Die pharmazeutischen Zusammensetzungen können geeignete Pufferstoffe wie Essigsäure in einem Salz, Citronensäure in einem Salz, Borsäure in einem Salz und Phosphorsäure in einem Salz enthalten.

Die pharmazeutischen Zusammensetzungen können auch gegebenenfalls geeignete Konservierungsstoffe wie Benzalkoniumchlorid, Chlorbutanol, Parabene und Thimerosal enthalten.

Die pharmazeutischen Zusammensetzungen werden gewöhnlich in einer einheitlichen Dosisform dargeboten und können in an sich bekannter Weise hergestellt werden. Pharmazeutische Zusammensetzungen können beispielsweise in Form von Kapseln, Tabletten, Lutschpastillen, Lösungen, Suspensionen, Sirupen, Elixieren oder als Emulsion vorliegen.

Zusammensetzungen, die für eine parenterale Verabreichung geeignet sind, umfassen gewöhnlich eine sterile wässrige oder nicht-wässrige Zubereitung des Wirkstoffs, die vorzugsweise mit dem Blut des Empfängers isotonisch ist. Verträgliche Träger und Lösungsmittel sind beispielsweise Ringer-Lösung und isotonische Natriumchloridlösung. Zusätzlich werden gewöhnlich sterile, fixierte Öle als Lösungs- oder Suspensionsmedium eingesetzt.

Die vorliegende Erfindung wird durch die nachstehenden Abbildungen und Beispiele ausführlich beschrieben, die ausschließlich der Erläuterung dienen und nicht begrenzend zu verstehen sind. Dem Fachmann sind aufgrund der Beschreibung und der Beispiele weitere Ausführungsformen zugänglich.

### Abbildungen:

**Abb. 1****: qPCR-Analyse von SEQ ID NO: 5 in Normal- und Tumorgeweben.** Quantitative Expressionsanalyse von SEQ ID NO: 5 in Normalgeweben und verschiedenen Tumoren (Pools aus jeweils 3-5 Einzelproben, rechte Seite).
   **A:** Logarithmische Darstellung der relativen Expression (-fache Aktivierung).
   **B:** Bild nach gelelektrophoretischer Auftrennung der amplifizierten Fragmente. Eine tumorselektive Expression ist ersichtlich.
**Abb. 2****: Detailanalyse der SEQ ID NO: 5-spezifischen Expression in Normal- und Tumorgeweben.**
   **A:** Quantitative Expressionsanalyse von SEQ ID NO: 5 in verschiedenen HNO-, Nieren- und Uterustumoren im Vergleich zur Expression in den dazugehörigen Normalgeweben. Logarithmische Darstellung.
   **B:** Bild nach gelelektrophoretischer Auftrennung der amplifizierten Fragmente. Eine tumorselektive Expression ist ersichtlich.

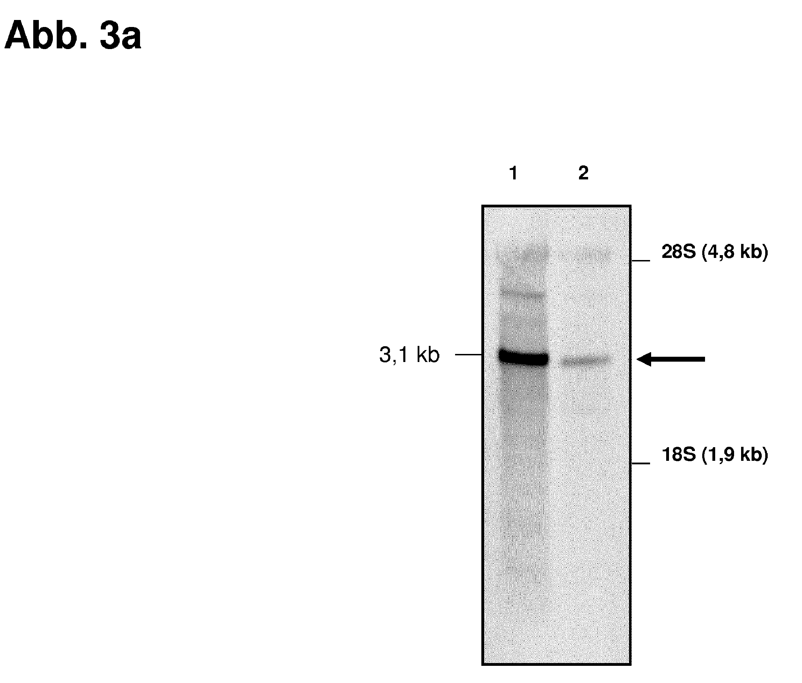

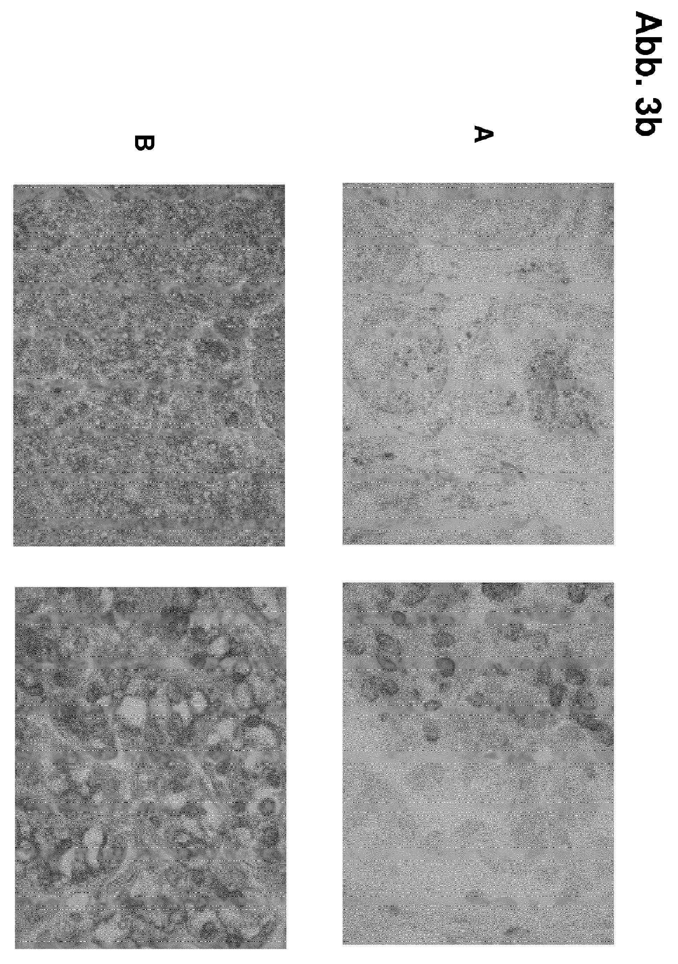

**Abb. 3a****: Northern-Blot-Analyse mit einer SEQ ID NO: 5-spezifischen Sequenz.** Hybridisierung einer DIG-markierten DNA-Sonde, die durch PCR-Amplifikation mit den Primern gemäß SEQ ID NO: 7 und 8 hergestellt wurden, mit Testis-spezifischer RNA. Spur 1: 2 µg Testis-spezifische RNA; Spur 2: 1 µg Testis-spezifische RNA.
**Abb. 3b****: Immunhistochemie mit SEQ ID NO: 5-spezifischem Antiserum.** Wie aus den RT-PCR-Reaktionen zu erwarten, konnte mit einem spezifischen Antiserum auch das Protein gemäß SEQ ID NO: 5 in Hodengewebe (**A**) als auch in Nierenzellkarzinomen (**B**) nachgewiesen werden.

### Beispiele:

### Material und Methoden

Die Begriffe "*in silico* ", und "elektronisch" beziehen sich rein auf die Nutzung von auf Datenbanken beruhenden Verfahren, mit denen auch Labor-experimentelle Vorgänge simuliert werden können.

Alle anderen Begriffe und Termini sind, falls nicht explizit anders definiert, so verwendet, wie sie der Fachmann versteht. Die genannten Techniken und Verfahren erfolgen in an sich bekannter Weise und sind z.B. in Sambrook et al., Molecular Cloning: A Laboratory Manual, 2. Auflage (1989), Cold Spring Harbor Laboratory Press, Cold Spring Harbor, N.Y. beschrieben. Alle Verfahren, die die Verwendung von Kits und Reagenzien einschließen, sind entsprechend den Angaben der Hersteller durchgeführt.

### A. Datamining-basierte Strategie zur Identifizierung von Tumor-assoziierten Antigenen

Es wurden öffentliche humane Protein- und Nukleinsäuredatenbanken im Hinblick auf krebsspezifische Antigene untersucht, die auf der Zelloberfläche zugänglich sind. Die Definition der dafür notwendigen Filterkriterien zusammen mit einer Hochdurchsatz-Methodik zur Analyse möglichst aller Proteine bildeten den zentralen Bestandteil dieser Strategie.

Den Ausgangspunkt bildeten die hauptsächlich aus dem humanen Genomprojekt vorhergesagten potenziellen Gene, die in der "RefSeq" Datenbank (Pruitt et al., Trends Genet. Jan;16(1):44-47, 2000) des "National Center for Biotechnology Information" (NCBI) als rein modellhafte Protein- (XP-) bzw. mRNA-Einträge (XM-) abgelegt sind. In einem weiteren Ansatz wurden auch die validierten Proteineinträge (NP-) bzw. die korrespondierenden mRNAs (NM-) derselben Datenbank in gleicher Weise analysiert. Dem Grundprinzip (hypothetisches) Gen zu mRNA zu Protein folgend wurden die Proteine unter Kombination mehrerer Prädiktionsprogramme für Proteinanalyse zunächst auf das Vorhandensein von Transmembrandomänen hin untersucht. Aus der humanen XP-Fraktion der "RefSeq" Datenbank wurden insgesamt 19.544 Einträge analysiert, wobei 2.025 hypothetische Proteine den Filterkriterien genügten. Die humane NP-Fraktion lieferte insgesamt 19.110 Einträge mit einem Anteil von 4.634 gefilterten Proteinen.

Die korrespondierende mRNA jedes dieser 2.025 bzw. 4.634 Proteine wurde anschließend einer Homologiesuche in der EST-Datenbank (Boguski et al., Nat. Genet. 4(4):332-333, 1993) des NCBI mit Hilfe des "BLAST" Algorithmus (Altschul et al., Nucleic Acids Res.25:3389-3402, 1997) unterzogen. Die Filterkriterien wurden bei dieser Suche stringent eingestellt. Insgesamt 1.270 hypothetische mRNAs erzielten dabei mindestens einen Treffer in der EST-Datenbank, wobei die Anzahl der Treffer in Einzelfällen mehr als 1.000 betrug.

Für jeden Einzelnen dieser validen Treffer wurde anschließend die gewebsspezifische Herkunft der zugrunde liegenden cDNA Bibliothek sowie der Name der Bibliothek ermittelt. Die daraus resultierenden Gewebe wurden in vier verschiedene Gruppen eingeteilt, die von dispensiblen Organen (Gruppe 3) bis hin zu absolut lebensnotwendigen Organen reichten (Gruppe 0). Eine weitere Gruppe 4 bildeten alle Proben, die aus Krebsgewebe gewonnen wurden. Die Verteilung der Treffer auf die fünf Gruppen wurde in einer Tabelle festgehalten, die nach dem besten Verhältnis der Summe der Gruppen 3 und 4 gegenüber der Summe der Gruppen 0-2 sortiert wurde. Dabei erreichten diejenigen mRNAs einen Spitzenplatz, deren EST Treffer ausschließlich Krebsgewebe entstammten, gefolgt von denjenigen, die darüber hinaus noch in Geweben dispensibler Organe der Gruppe 3 zu finden sind. Ein weiteres Kriterium für die Aussagekraft dieser Verteilung bildete die Anzahl der unabhängigen cDNA-Bibliotheken, aus denen die ESTs gewonnen wurden und die in einer eigenen Spalte in der Tabelle festgehalten wurde.

Da es sich bei den im ersten Ansatz ermittelten Transkripten und den korrespondierenden Proteinen zunächst um hypothetische Konstrukte handelt, wurden noch weitere Filterkriterien hinzugezogen, die die reale Existenz der mRNAs und damit auch der Proteine belegen sollten. Dazu wurde jede mRNA mit Hilfe des Programms "Spidey" (Wheelan et al., Genome Res. 11(11):1952-1957, 2001) dem vorhergesagten Genlokus verglichen. Nur diejenigen Transkripte, die mindestens einen Spleißvorgang aufweisen, d.h. die sich auf mindestens 2 Exons verteilen, wurden für weitergehende Analysen verwendet.

Die sequenzielle Anwendung aller genannten Filter führte zu den hierin beschriebenen Tumor-assoziierten Antigenen, die aufgrund einer vorhergesagten Transmembrandomäne und der damit verbundenen Topologie als von extrazellulär zugänglich anzusehen sind. Das aus den EST-Daten abgeleitete Expressionsprofil weist in allen Fällen auf eine krebsspezifische Expression hin, die sich höchstens noch auf dispensible Organe erstrecken kann.

### B. Validierungsstrategie der durch in silico Analyse identifizierten Tumor-assoziierten Antigene

Zur Nutzung der Targets für immuntherapeutische Zwecke (Antikörpertherapie mittels monoklonaler Antikörper, Vakzinierung, T-Zell Rezeptor-vermittelte therapeutische Ansätze; vgl. EP-B-0 879 282) oder andere zielgerichtete Ansätze (small compounds, siRNA etc.) bei der Krebstherapie sowie für diagnostische Fragestellungen ist die Validierung der gemäß der hierin beschriebenen Strategie identifizierten Targets von zentraler Bedeutung. Die Validierung erfolgt dabei durch Expressionsanalyse sowohl auf RNA als auch auf Proteinebene.

### 1. Untersuchung der RNA Expression

Die erste Charakterisierung der identifizierten Tumorantigene erfolgt mit Hilfe von RNA, die aus verschiedenen Geweben bzw. aus gewebespezifischen Zelllinien gewonnen wird. Weil das differentielle Expressionsmuster aus gesundem Gewebe im Vergleich zu Tumorgewebe eine entscheidende Bedeutung für die spätere therapeutische Anwendung hat, erfolgt die Charakterisierung der Zielgene bevorzugt mit Hilfe dieser Gewebeproben.

Die Isolierung von Gesamt-RNA aus nativen Gewebeproben oder aus Tumorzelllinien erfolgt mit Verfahren, die in der Molekularbiologie Standard sind. Zum Beispiel kann die Isolierung mit Hilfe des RNeasy Maxi Kits (Qiagen, Kat. Nr. 75162) nach Vorschrift durch den Hersteller erfolgen. Dieses Isolierungsverfahren beruht auf der Verwendung von Guanidiniumisothiocyanat als chaotropes Reagenz. Alternativ kann die Isolierung mit saurem Phenol durchgeführt werden (Chomczynski & Sacchi, Anal. Biochem. 162: 156-159, 1987). Nach Aufarbeitung des Gewebes mittels Guanidiniumisothiocyanat wird die RNA mit saurem Phenol extrahiert, anschließend die RNA mit Isopropanol gefällt und in DEPC-behandeltes Wasser aufgenommen.

2-4 µg der so isolierten RNA werden anschließend z.B. mittels Superscript II (Invitrogen) entsprechend dem Protokoll des Herstellers in cDNA umgeschrieben. Das Priming der cDNA Synthese erfolgt dabei mit Hilfe von zufälligen Hexameren (z.B. Roche Diagnostics) nach Standardprotokollen des jeweiligen Herstellers. Zur Qualitätskontrolle werden die cDNAs mit Primern in 30 Zyklen amplifiziert, die spezifisch für das nur gering exprimierte p53 Gen sind. Nur p53 positive cDNA Proben werden für die weiteren Reaktionsschritte verwendet.

Zur detaillierten Analyse der Targets wird auf Basis eines cDNA-Archivs, das aus verschiedenen Normal- und Tumorgeweben sowie aus Tumorzelllinien isoliert wurde, eine Expressionsanalyse mittels PCR bzw. quantitativer PCR (qPCR) durchgeführt. Dazu werden 0,5 µl cDNA aus dem obigen Ansatz mit einer DNA-Polymerase (z.B. 1 U HotStarTaq DNA-Polymerase, Qiagen) analog den Protokollen des jeweiligen Herstellers amplifiziert (Gesamtvolumen des Ansatzes: 25-50 µl). Neben der Polymerase enthält der Amplifikationsansatz 0,3 mM dNTPs, Reaktionspuffer (Endkonzentration 1 x, abhängig vom Hersteller der DNA-Polymerase) und je 0,3 mM des gen-spezifischen "sense"- und "antisense"-Primers.

Die spezifischen Primer des Zielgens werden, soweit möglich, so ausgewählt, das sie in zwei unterschiedlichen Exons liegen und somit genomische Kontaminationen nicht zu falsch positiven Ergebnissen führen. Bei einer nicht quantitativen Endpunkt-PCR wird die cDNA typischerweise 15 Minuten bei 95°C inkubiert, um die DNA zu denaturieren und um das Hot-Start-Enzyms zu aktivieren. Anschließend wird die DNA in 35 Zyklen amplifiziert (1 min 95°C, 1 min Primer spezifische Hybridisierungstemperatur (ca. 55-65°C), 1 min 72°C zur Elongation der Amplifikate). 10 µl des PCR Ansatzes werden anschließend auf Agarosegelen aufgetragen und im elektrischen Feld aufgetrennt. Durch Färben mit Ethidiumbromid wird die DNA in den Gelen sichtbar gemacht und das Ergebnis der PCR durch ein Foto dokumentiert.

Alternativ zur konventionellen PCR kann die Expressionsanalyse eines Zielgens auch durch quantitative real time PCR erfolgen. Zu dieser Analyse sind inzwischen verschiedene Analysesysteme erhältlich, die bekanntesten sind das ABI PRISM Sequence detection system (TaqMan, Applied Biosystems), der iCycler (Biorad) sowie der Light cycler (Roche Diagnostics). Wie oben beschrieben wird ein spezifischer PCR Ansatz einem Lauf in den "real time"-PCR-Geräten unterzogen. Durch Zusatz eines DNA interkalierenden Farbstoffes (z.B Ethidiumbromid, CybrGreen) wird die neu synthetisierte DNA durch spezifische Lichtanregung (nach Angaben der Farbstoffhersteller) sichtbar gemacht. Durch eine Vielzahl von Messpunkten während der Amplifikation kann der gesamte Prozess verfolgt und eine quantitative Bestimmung der Nukleinsäurekonzentration des Zielgens durchgeführt werden. Die Normalisierung des PCR Ansatzes erfolgt durch Messung eines "housekeeping Gens" (z.B. 18S RNA, ß-Actin). Alternative Strategien über Fluoreszenz-markierte DNA-Sonden erlauben ebenfalls die quantitative Bestimmung des Zielgens aus einer spezifischen Gewebeprobe (siehe TaqMan Applikationen der Fa. Applied Biosystems).

### 2. Klonierung

Die Klonierung des gesamten Zielgens, die für die weitere Charakterisierung des Tumorantigens notwendig ist, erfolgt nach gängigen molekularbiologischen Verfahren (z.B. in "Current Protocols in Molecular Biology", John Wiley & Sons Ltd., Wiley InterScience). Zur Klonierung bzw. Sequenzanalyse des Zielgens wird dieses zunächst mit einer DNA-Polymerase mit "proof reading Funktion" (z.B. pfu, Roche Diagnostics) amplifiziert. Das Amplifikat wird anschließend mit Standardverfahren in einen Klonierungsvektor ligiert. Positive Klone werden durch Sequenzanalyse identifiziert und anschließend mit Hilfe von Prädiktionsprogrammen und bekannten Algorithmen charakterisiert.

### 3. Prädiktion des Proteins

Viele gemäß der hierin beschriebenen Strategie gefundene Gene (insbesondere aus der XM Domäne der RefSeq) sind Gen-Neuentdeckungen, für die das Volllänge-Gen kloniert, das offene Leseraster ermittelt und die Proteinsequenz abgeleitet und analysiert werden muss. Für die Volllängeklonierung der Sequenz haben wir gängige Protokolle zur "Rapid amplification of cDNA ends", sowie Screening von cDNA Expressionsbanken mit gen-spezifischen Sonden verwendet (Sambrook et al., Molecular Cloning: A Laboratory Manual, 2. Auflage (1989), Coli Spring Harbor Laboratory Press, Coli Spring Harbor, N. Y*.*). Nach Zusammensetzung der so gefundenen Fragmente wurden potentielle Offene Leseraster (ORF) durch gängige Prädiktionsprogramme prädiziert. Da durch die Position des PolyA-Schwanzes und Polyadenylierungs-Motiven die Orientierung des potentiellen Genproduktes vorgegeben wird, verbleiben von möglichen 6 Leserastern nur noch die 3 der jeweiligen Orientierung. Oft ergibt sich aus diesen nur ein hinreichend großes offenes Leseraster, das für ein Protein kodieren kann, während die anderen Leseraster zu viele Stop-Codons aufweisen und für kein realistisches Proteine kodieren würden. Bei alternativen offenen Leserastern unterstützt die Berücksichtigung der Kozak-Kriterien für optimale Transkriptions-Initiierung sowie die Analyse der sich potentiell ergebenden abgeleiteten Proteinsequenzen die Identifizierung des authentischen ORF. Dies wird weiter verifiziert durch Generierung von Immunseren gegen abgeleitete Proteine der potentiellen ORFs und ihre Analyse auf Erkennung eines realen Proteins in Geweben und Zelllinien.

### 4. Gewinnung von Antikörpern

Die Charakterisierung der gemäß der hierin beschriebenen Strategie identifizierten Tumor- assoziierten Antigene erfolgt beispielsweise durch die Verwendung von Antikörpern. Ferner umfasst die Erfindung die diagnostische Verwendung von Antikörpern und es wird auch die therapeutische Anwendung von Antikörpern beschrieben. Dabei können Antikörper Proteine in nativem und/oder denaturierten Zustand erkennen (Anderson et al., J. Immunol. 143: 1899-1904, 1989; Gardsvoll, J. Immunol. Methods 234: 107-116, 2000; Kayyem et al., Eur. J. Biocherm. 208: 1-8, 1992; Spiller et al., J. Immunol. Methods 224: 51-60, 1999).

Antiseren, die spezifische Antikörper enthalten, die an das Zielprotein spezifisch binden, können über verschiedene Standardverfahren hergestellt werden; vgl. beispielsweise "Monoclonal Antibodies: A Practical Approach" von Philip Shepherd, Christopher Dean ISBN 0-19-963722-9, "Antibodies: A Laboratory Manual" von Ed Harlow, David Lane ISBN: 0879693142 und "Using Antibodies: A Laboratory Manual: Portable Protocol NO" von Edward Harlow, David Lane, Ed Harlow ISBN: 0879695447. Dabei ist auch möglich, affine und spezifische Antikörper zu generieren, die komplexe Membranproteine in ihrer nativen Form erkennen (Azorsa et al., J. Immunol. Methods 229: 35-48, 1999; Anderson et al., J. Immunol. 143: 1899-1904, 1989; Gardsvoll, J. Immunol. Methods. 234: 107-116, 2000). Dies ist vor allem für die Herstellung von therapeutischen Antikörpern von Bedeutung, , hat aber auch für viele diagnostische Anwendungen eine hohe Bedeutung. Dazu kann sowohl mit dem gesamten Protein als auch mit extrazellulären Teilsequenzen immunisiert werden.

### Immunisierung und Gewinnung von polyklonalen Antikörpern

Verschiedenste Immunisierungsprotokolle sind publiziert. Eine Spezies (z.B. Kaninchen, Mäuse) wird durch eine erste Injektion des gewünschten Zielproteins immunisiert. Durch eine zweite oder dritte Immunisierung innerhalb eines definierten Zeitraums (ca. 2-4 Wochen nach der letzten Immunisierung) lässt sich die Immunantwort des Tieres gegen das Immunogen verstärken. Wiederum nach verschiedenen definierten Zeitabständen (1. Blutung nach 4 Wochen, anschließend alle 2-3 Wochen bis zu 5 Entnahmen) wird den Tieren Blut entnommen und Immunserum gewonnen. Die so entnommenen Immunseren enthalten polyklonalen Antikörper, mit denen das Zielprotein im Western Blot, durch die Durchflusszytometrie, Immunfluoreszenz oder Immunhistochemie nachgewiesen und charakterisiert werden kann.

Die Immunisierung der Tiere erfolgt in der Regel über eines von vier gut etablierten Verfahren, wobei auch andere Verfahren existieren. Immunisiert werden kann dabei mit für das Zielprotein spezifischen Peptiden, dem gesamten Protein, mit extrazellulären Teilsequenzen eines Proteins, das experimentell oder über Prädiktionsprogramme identifiziert werden kann. Da die Prädiktionsprogramme nicht immer fehlerfrei arbeiten, wird u.U. auch mit zwei Domänen gearbeitet, die voneinander durch eine Transmembrandomäne getrennt sind. Eine der beiden Domänen muss dann extrazellulär sein, was dann experimentell belegt werden kann (siehe nachstehend). Die Durchführung einer Immunisierung wird als Service von verschiedenen Dienstleistern kommerziell angeboten.
(1) Im ersten Fall werden Peptide (Länge: 8-12 Aminosäuren) über *in vitro* Verfahren synthetisiert (durch einen kommerziellen Service möglich) und diese Peptide zur Immunisierung verwendet. In der Regel erfolgen 3 Immunisierungen (z.B. mit einer Konzentration von 5-100 µg/Immunisierung).
(2) Alternativ kann die Immunisierung durch rekombinante Proteine erfolgen. Dazu wird die klonierte DNA des Zielgens in einen Expressionsvektor kloniert und das Zielprotein analog den Bedingungen des jeweiligen Herstellers (z.B. Roche Diagnostics, Invitrogen, Clontech, Qiagen) z.B. zellfrei *in vitro,* in Bakterien (z.B. *E. coli*), in Hefe (z.B. *S. pombe*), in Insektenzellen oder in Säugetierzellen synthetisiert. Dabei ist auch die Synthese des Zielproteins mit Hilfe von viralen Expressionssystemen möglich (z.B. Baculovirus, Vacciniavirus, Adenovirus). Nach Synthese in einem der Systeme wird das Zielprotein aufgereinigt. Die Aufreinigung erfolgt dabei in der Regel über chromatographische Verfahren. Dabei können auch Proteine für die Immunisierung verwendet werden, die über einen molekularen Anker als Hilfsmittel zur Reinigung verfügen (z.B. His-Tag, Qiagen; FLAG-Tag, Roche Diagnostics; GST-Fusionsproteine). Eine Vielzahl von Protokollen befindet sich z.B. in den "Current Protocols in Molecular Biology", John Wiley & Sons Ltd., Wiley InterScience. Nach Reinigung des Zielproteins erfolgt eine Immunisierung wie vorstehend beschrieben.
(3) Falls eine Zelllinie zur Verfügung steht, die das gewünschte Protein endogen synthetisiert, kann auch diese Zelllinie direkt zur Herstellung des spezifischen Antiserums verwendet werden. Die Immunisierung erfolgt dabei in 1-3 Injektionen mit jeweils ca. 1-5 x 10⁷ Zellen.
(4) Die Immunisierung kann auch durch Injektion von DNA (DNA-Immunisierung) erfolgen. Dazu wird das Zielgen zunächst in einen Expressionsvektor kloniert, so dass die Zielsequenz unter der Kontrolle eines starken eukaryontischen Promotors steht (z.B. CMV-Promotor). Anschließend wird DNA (z.B. 1-10 µg pro Injektion) als Immunogen mit einer "gene gun" in stark durchblutete, kapillare Bereiche eines Organismus transferiert (z.B. Maus, Kaninchen). Die transferierte DNA wird von Zellen des Tieres aufgenommen, das Zielgen wird exprimiert und das Tier entwickelt schließlich eine Immunantwort gegen das Zielprotein (Jung et al., Mol. Cells 12: 41-49, 2001; Kasinrerk et al., Hybrid Hybridomics 21: 287-293, 2002).

### Gewinnung monoklonaler Antikörper

Monoklonale Antikörper werden traditionell mit Hilfe der Hybridoma Technologie hergestellt (Technische Details: siehe "Monoclonal Antibodies: A Practical Approach" von Philip Shepherd, Christopher Dean ISBN 0-19-963722-9; "Antibodies: A Laboratory Manual" von Ed Harlow, David Lane ISBN: 0879693142, "Using Antibodies: A Laboratory Manual: Portable Protocol NO" von Edward Harlow, David Lane, Ed Harlow ISBN: 0879695447). Als ein neues Verfahren wird auch die so genannte "SLAM" Technologie eingesetzt. Hierbei werden B-Zellen aus Vollblut isoliert und die Zellen monoklonalisiert. Anschließend wird der Überstand der vereinzelten B-Zelle auf ihre Antikörperspezifität hin analysiert. Im Gegensatz zur Hybridomatechnologie wird anschließend die variable Region des Antikörpergens durch eine Einzelzell-PCR amplifiziert und in einen geeigneten Vektor kloniert. Auf diese Art und Weise wird die Gewinnung von monoklonalen Antikörpern beschleunigt (de Wildt et al. J. Immunol. Methods 207:61-67, 1997).

### 5. Validierung der Targets mit proteinchemischen Verfahren unter Verwendung von Antikörpern

Mit den Antikörpern, die wie vorstehend beschrieben herstellbar sind, lassen sich eine Reihe von wichtigen Aussagen zu dem Targetprotein treffen. Im Einzelnen sind die nachstehenden Analysen zur Validierung des Zielproteins sinnvoll:

### Spezifität des Antikörpers

Um zu zeigen, dass ein Antikörper spezifisch nur an das gewünschte Zielprotein bindet, eignen sich am besten auf Zellkultur-basierende Tests mit anschließendem Western Blot (verschiedene Variationen sind z.B. in "Current Protocols in Proteinchemistry", John Wiley & Sons Ltd., Wiley InterScience, beschrieben). Für den Nachweis werden Zellen mit einer cDNA für das Zielprotein transfiziert, die unter Kontrolle eines starken eukaryontischen Promotors steht (z.B. Cytomegalievirus-Promotor; CMV). Zur Transfektion von Zelllinien mit DNA sind die verschiedensten Verfahren (z.B. Elektroporation, auf Liposomen basierende Transfektion, Kalziumphosphatpräzipitation) gut etabliert (z.B. Lemoine et al., Methods Mol. Biol. 75: 441-7, 1997). Alternativ können auch Zelllinien verwendet werden, die das Zielgen endogen exprimieren (Nachweis über Zielgen-spezifische RT-PCR). Zur Kontrolle werden im Experiment im Idealfall homologe Gene mit transfiziert, um im folgenden Western Blot die Spezifität des analysierten Antikörpers nachweisen zu können.

Im anschließenden Western Blot werden Zellen aus Zellkultur oder Gewebeproben, die das Zielprotein enthalten könnten, in einer 1%igen SDS Lösung lysiert und die Proteine dabei denaturiert. Die Lysate werden auf 8-15%igen denaturierenden Polyacrylamidgelen (enthalten 1% SDS) der Größe nach elekrophoretisch aufgetrennt (SDS-Polyacrylamid Gelelektrophorese, SDS-PAGE). Anschließend werden die Proteine durch eines von mehreren Blotting Verfahren (z.B. semi-dry Elektroblot; Biorad) auf eine spezifische Membran transferiert (z.B. Nitrozellulose, Schleicher & Schüll). Auf dieser Membran kann das gewünschte Protein sichtbar gemacht werden. Dazu wird die Membran zunächst mit dem Antikörper, der das Zielprotein erkennt (Verdünnung ca. 1:20-1:200, je nach Spezifität des Antikörpers), für 60 Minuten inkubiert. Nach einem Waschschritt wird die Membran mit einem zweiten, mit einem Marker (z.B. Enzyme wie Peroxidase oder alkalische Phosphatase) gekoppelten Antikörper inkubiert, der den ersten Antikörper erkennt. In einer Farb- oder chemilumineszenten Reaktion kann anschließend das Zielprotein auf der Membran sichtbar gemacht werden (z.B. ECL, Amersham Bioscience). Ein Antikörper mit einer hohen Spezifität für das Zielprotein sollte im Idealfall nur das gewünschte Protein selbst erkennen.

### Lokalisation des Zielproteins

Zur Bestätigung der im "in silico"-Ansatz identifizierten Membranlokalisation des Zielproteins werden verschiedene Verfahren verwendet. Ein wichtiges und gut etabliertes Verfahren unter Verwendung der vorstehend beschriebenen Antikörper ist die Immunfluoreszenz (IF). Dazu werden Zellen etablierter Zelllinien benutzt, die entweder das Zielprotein synthetisieren (Nachweis der RNA in der RT-PCR oder des Proteins im Western Blot) oder aber mit Plasmid-DNA transfiziert worden sind. Zur Transfektion von Zelllinien mit DNA sind die verschiedensten Verfahren (z.B. Elektroporation, auf Liposomen basierende Transfektion, Kalziumphosphatpräzipitation) gut etabliert (z.B. Lemoine et al., Methods Mol. Biol. 75: 441-7, 1997). Das in die Zellen transfizierte Plasmid kann bei der Immunfluoreszenz das unmodifizierte Protein kodieren oder aber auch unterschiedliche Aminosäuremarker an das Zielprotein koppeln. Die wichtigsten Marker sind z.B. das fluoreszierende "green fluorescent protein" (GFP) in seinen verschiedenen differentiell fluoreszierenden Formen, kurze Peptidsequenzen von 6-12 Aminosäuren, für die hoch affine und spezifische Antikörper zur Verfügung stehen, oder die kurze Aminosäuresequenz Cys-Cys-X-X-Cys-Cys, die über ihre Cysteine spezifische fluoreszierende Substanzen binden kann (Invitrogen). Zellen, die das Zielprotein synthetisieren, werden z.B. mit Paraformaldehyd oder Methanol fixiert. Anschließend können die Zellen bei Bedarf durch Inkubation mit Detergenzien (z.B. 0,2% Triton X-100) permeabilisiert werden. Anschließend werden die Zellen mit einem primären Antikörper inkubiert, der gegen das Zielprotein oder gegen einen der gekoppelten Marker gerichtet ist. Nach einem Waschschritt wird der Ansatz mit einem zweiten, mit einem fluoreszierenden Marker (z.B. Fluorescein, Texas Red, Dako) gekoppelten Antikörper inkubiert, der an den ersten Antikörper bindet. Anschließend werden die so markierten Zellen mit Glycerin überschichtet und mit Hilfe eines Fluoreszenzmikroskops nach den Angaben des Herstellers analysiert. Spezifische Fluoreszenzemissionen werden dabei, abhängig von den eingesetzten Substanzen, durch spezifische Anregung erreicht. Die Analyse erlaubt in der Regel die sichere Lokalisation des Zielproteins, wobei zur Bestätigung der Antikörperqualität und des Zielproteins in Doppelfärbungen zusätzlich zum Zielprotein auch die gekoppelten Aminosäuremarker oder andere Markerproteine angefärbt werden, deren Lokalisation bereits in der Literatur beschrieben ist. Ein Sonderfall stellt das GFP und seine Derivate dar, die direkt angeregt werden können und selbst fluoreszieren. Die Membranpermeabilität, die durch den Einsatz von Detergenzien gesteuert werden kann, erlaubt in der Immunfluoreszenz die Demonstration, ob ein immunogenes Epitop innerhalb oder außerhalb der Zelle lokalisiert ist. Die Prädiktion der ausgewählten Proteine kann so experimentell untermauert werden. Alternativ kann der Nachweis von extrazellulären Domänen mittels Durchflusszytometrie erfolgen. Dazu werden Zellen unter nicht permeabilisierenden Bedingungen (z.B. mit PBS/Na-Azid/2% FCS/ 5 mM EDTA) fixiert und im Durchflusszytometer nach Angaben des Herstellers analysiert. Nur extrazelluläre Epitope können bei diesem Verfahren von dem zu analysierenden Antikörper erkannt werden. Im Unterschied zu Immunfluoreszenz kann durch Verwendung von z.B. Propidiumiodid oder Trypanblau zwischen toten und lebenden Zellen unterschieden werden und damit falsch positive Ergebnisse vermieden werden.

Ein weiterer wichtiger Nachweis erfolgt durch die Immunhistochemie (IHC) an spezifischen Gewebeproben. Ziel dieses Verfahrens ist es, die Lokalisation eines Proteins in einem funktionell intakten Gewebeverband zu identifizieren. Die IHC dient im Einzelnen dazu, um (1) die Menge an Zielprotein in Tumor- und Normalgeweben abschätzen zu können, (2) zu analysieren, wie viele Zellen in Tumor- und gesundem Gewebe das Zielgen synthetisieren, und (3) den Zelltyp in einem Gewebe (Tumor, gesunde Zellen) zu definieren, in dem das Zielprotein nachweisbar ist. Alternativ können die Proteinmengen eines Zielgens durch Gewebsimmunfluoreszenz mittels Digitalkamera und geeigneter Software (z.B. Tillvision, Till-photonics, Deutschland) quantifiziert werden. Die Technologie ist häufig publiziert worden, Details für Färbung und Mikroskopie sind daher z.B. "Diagnostic Immunohistochemistry" von David J., MD Dabbs ISBN: 0443065667 oder in "Microscopy, Immunohistochemistry, and Antigen Retrieval Methods: For Light and Electron Microscopy" ISBN: 0306467704 zu entnehmen. Zu beachten ist, dass aufgrund der Eigenschaften von Antikörpern unterschiedliche Protokolle verwendet werden müssen (nachstehend ist ein Beispiel beschrieben), um zu einem aussagekräftigen Ergebnis zu kommen.

In der Regel werden histologisch definierte Tumorgewebe und als Referenz vergleichbare gesunde Gewebe in der IHC eingesetzt. Als Positiv- und Negativkontrollen können dabei auch Zelllinien dienen, bei denen die Präsenz des Zielgens durch RT-PCR Analysen bekannt ist. Eine Hintergrundkontrolle ist immer mitzuführen.

Formalin-fixierte (ein anderes Fixierungsverfahren mit z.B. Methanol ist auch möglich) und in Paraffin eingebettete Gewebestücke mit einer Dicke von 4µm werden auf einem Glasträger aufgebracht und z.B. mit Xylol deparaffiniert. Die Proben werden mit TBS-T gewaschen und in Serum blockiert. Anschließend erfolgt die Inkubation mit dem ersten Antikörper (Verdünnung: 1:2 bis 1:2000) für 1-18 Stunden, wobei in der Regel affinitätsgereinigete Antikörper verwendet werden. Nach einem Waschschritt erfolgt eine ca. 30-60 minütige Inkubation mit einem zweiten Antikörper, der mit einer Alkalischen Phosphatase (alternativ: z.B. Peroxidase) gekoppelt und gegen den ersten Antikörper gerichtet ist. Anschließend erfolgt eine Farbreaktion unter Verwendung der Alkalischen Phosphatase (vgl. beispielsweise Shi et al., J. Histochem. Cytochem. 39: 741-748, 1991; Shin et al., Lab. Invest. 64: 693-702, 1991). Zum Nachweis der Antikörper-Spezifität kann die Reaktion durch vorherige Zugabe des Immunogens kompetitiert werden.

### Analyse von Proteinmodifikationen

Sekundäre Proteinmodifikationen wie zum Beispiel N- und O-Glykosylierungen oder Myristylierungen können die Zugänglichkeit von immunogenen Epitopen behindern oder sogar ganz verhindern und damit die Wirksamkeit von Antikörpertherapien in Frage stellen. Zudem konnte vielfach nachgewiesen werden, dass sich Art und Menge der sekundären Modifikationen in Normal- und Tumorgewebe unterscheiden (z.B. Durand & Seta, 2000; Clin. Chem. 46: 795-805; Hakomori, 1996; Cancer Res. 56: 5309-18). Die Analyse dieser Modifikationen ist daher essentiell für den Therapieerfolg eines Antikörpers. Potentielle Bindestellen lassen sich durch spezifische Algorithmen prädizieren.

Die Analyse von Proteinmodifikationen erfolgt in der Regel im Western Blot (siehe vorstehend). Vor allem Glykosylierungen, die in der Regel eine Größe von mehreren kDa haben, führen zu einer größeren Gesamtmasse des Zielproteins, die sich in der SDS-PAGE auftrennen lässt. Zum Nachweis von spezifischen O- und N-glycosidischen Bindungen werden Proteinlysate vor der Denaturierung durch SDS mit O- oder N- Glykosylasen inkubiert (nach Angaben des jeweiligen Herstellers, z.B. PNgase, Endoglykosidase F, Endoglykosidase H, Roche Diagnostics). Anschließend erfolgt ein Western Blot wie vorstehend beschrieben. Bei Verringerung der Größe eines Zielproteins kann so nach Inkubation mit einer Glykosidase eine spezifische Glykosylierung nachgewiesen und auf diesem Weg auch die Tumorspezifität einer Modifikation analysiert werden.

### Funktionsanalyse des Zielgens

Die Funktion des Zielmoleküls kann entscheidend für seinen therapeutischen Nutzen sein, so dass funktionelle Analysen ein wichtiger Baustein bei der Charakterisierung von therapeutisch nutzbaren Molekülen sind. Die Funktionsanalyse kann entweder in Zellen in Zellkulturexperimenten oder aber in vivo mit Hilfe von Tiermodellen erfolgen. Dabei wird das Gen des Zielmoleküls entweder durch Mutation ausgeschaltet ("knockout") oder aber die Zielsequenz in die Zelle bzw. den Organismus eingefügt ("knockin"). Man kann so funktionelle Veränderungen im zellulären Kontext einerseits durch den Funktionsverlust des zu analysierenden Genes ("loss of function") analysieren. Im zweiten Fall lassen sich Veränderungen analysieren, die durch die Ergänzung des analysierten Genes verursacht werden ("gain of function").

### a. Funktionsanalyse in Zellen

Transfektion. Zur Analyse des "gain of function" muss das Gen des Zielmoleküls in die Zelle transferiert werden. Dazu werden Zellen mit einer DNA transfiziert, die die Synthese des Zielmoleküls erlauben. In der Regel steht das Gen des Zielmoleküls dabei unter Kontrolle eines starken eukaryontischen Promotors (z.B. Cytomegalievirus-Promotor; CMV). Zur Transfektion von Zelllinien mit DNA sind die verschiedensten Verfahren (z.B. Elektroporation, auf Liposomen basierende Transfektion, Kalziumphosphatpräzipitation) gut etabliert (z.B. Lemoine et al., Methods Mol. Biol. 75: 441-7, 1997). Das Gen kann dabei entweder ohne genomische Integration transient oder aber mit genomischer Integration nach Selektion mit z.B. Neomycin stabil synthetisiert werden.

RNA interference (siRNA). Eine Expressionsinhibition des Zielgens, die unter Umständen einen vollständigen Funktionsverlust des Zielmoleküls in Zellen induziert, kann durch die "RNA interference" (siRNA) Technologie in Zellen erzeugt werden (Hannon, GJ. 2002. RNA interference. Nature 418: 244-51; Czauderna et al. 2003. Nucl. Acid Res. 31: 670-82). Dazu werden Zellen mit kurzen, ca. 20-25 Nukleotide langen, doppelsträngigen RNA Molekülen transfiziert, die für das Zielmolekül spezifisch sind. Ein enzymatischer Prozess führt anschließend zum Abbau der spezifischen RNA des Zielgens, was in einer verminderten Expression des Zielproteins resultiert, und ermöglicht damit die funktionelle Analyse des Zielgens.

Zelllinien, die mittels Transfektion oder siRNA modifiziert wurden, können anschließend auf unterschiedliche Art und Weise analysiert werden. Nachstehend sind die geläufigsten Beispiele aufgeführt.

### 1. Proliferation und Zellzyklusverhalten

Eine Vielzahl von Verfahren ist zur Analyse der Zellproliferation etabliert und wird von verschiedenen Unternehmen kommerziell angeboten (z.B. Roche Diagnostics, Invitrogen, Details zu den Testverfahren sind in den zahlreichen Applikationsprotokollen beschrieben). Die Zellzahl in Zellkulturexperimenten lässt sich durch einfaches Auszählen oder durch kolometrische Tests ermitteln, die die metabolische Aktivität der Zellen messen (z.B. wst-1, Roche Diagnostics). Metabolische Testverfahren messen indirekt über enzymatische Marker die Zellzahl in einem Experiment. Direkt kann die Zellproliferation durch Analyse der DNA Syntheserate z.B. durch Zugabe von Bromdesoxyuridin (BrdU) gemessen werden, der Nachweis des integrierten BrdU erfolgt über spezifische Antikörper kolometrisch.

### 2. Apoptose und Zytotoxizität

Eine große Anzahl von Testsystemen zum Nachweis von zellulärer Apoptose und von Zytotoxizität ist verfügbar. Ein entscheidendes Charakteristikum ist die spezifische, enzymabhängige Fragmentierung der genomischen DNA, die irreversibel ist und sicher zum Tod der Zelle führt. Verfahren zum Nachweis dieser spezifischen DNA Fragmente sind kommerziell erhältlich. Als zusätzliches Verfahren steht der "TUNEL assay" zur Verfügung, der DNA Einzelstrangbrüche auch in Gewebeschnitten nachweisen kann. Zytotoxizität wird vor allem über eine veränderte Zellpermeabilität nachgewiesen, die als Marker für den Vitalitätszustand von Zellen dient. Dazu werden entweder im Zellkulturüberstand Marker analysiert, die normalerweise intrazellulär zu finden sind. Alternativ kann auch die Aufnahmefähigkeit von Farbmarkern analysiert werden, die von intakten Zellen nicht aufgenommen werden. Die bekanntesten Beispiele für Farbmarker sind Trypanblau und Propidiumiodid, ein üblicher intrazellulärer Marker ist die Laktatdehydrogenase, die im Überstand enzymatisch nachgewiesen werden kann. Unterschiedliche Testsysteme stehen von verschiedenen kommerziellen Anbietern (z.B. Roche Diagnostics, Invitrogene) zur Verfügung.

### 3. Migrationsassay

Die Fähigkeit von Zellen zur Migration wird in einem spezifischen Migrationstest vorzugsweise mit Hilfe einer Boyden Kammer (Corning Costar) analysiert (Cinamon G., Alon R. J. Immunol. Methods. 2003 Feb; 273(1-2):53-62; Stockton et al. 2001. Mol. Biol. Cell. 12: 1937-56). Dazu werden Zellen auf einem Filter mit spezifischer Porengröße kultiviert. Zellen, die migrieren können, sind in der Lage, durch diesen Filter in ein weiteres darunter liegendes Kulturgefäß zu wandern. Eine anschließende mikroskopische Analyse erlaubt dann die Bestimmung eines möglicherweise veränderten Migrationsverhaltens, dass durch den "gain of function" bzw. "loss of function" des Zielmoleküls induziert wurde.

### b. Funktionsanalyse in Tiermodellen

Alternativ zu Zellkulturexperimenten bieten sich zur Analyse der Zielgenfunktion aufwendige in vivo Experimente in Tiermodellen an. Diese Modelle haben im Vergleich zu den zellbasierenden Verfahren den Vorteil, dass sie Fehlentwicklungen bzw. Krankheiten nachweisen können, die erst im Kontext des gesamten Organismus nachweisbar sind. Eine Vielzahl von Modellen für humane Erkrankungen sind inzwischen verfügbar (Abate-Shen & Shen. 2002. Trends in Genetics S 1-5; Matsusue et. al. 2003. J. Clin. Invest. 111:737-47). Verschiedene Tiermodelle wie zum Beispiel Hefe, Nematoden oder Zebrafische sind inzwischen intensiv charakterisiert worden. Bevorzugte Modelle sind aber im Vergleich zu anderen Spezies mammale Tiermodelle wie zum Beispiel die Maus (Mus musculus), weil sie die biologischen Prozesse im humanen Kontext am besten abbilden können. Für Mäuse sind in den letzten Jahren sowohl transgene Verfahren etabliert worden, die neue Gene in das Mausgenom integrieren ("gain of function` ; Jegstrup I. et al. 2003. Lab Anim. 2003 Jan.;37(1):1-9). Alternativ werden durch andere methodische Ansätze Gene im Mausgenom ausgeschaltet und so ein Funktionsverlust eines gewünschten Gens induziert (knockout Modelle, "loss of function"; Zambrowicz BP & Sands AT. 2003. Nat. Rev. Drug Discov. 2003 Jan;2(1):38-51; Niwa H. 2001. Cell Struct. Funct. 2001 Jun;26(3):137-48.); technische Details sind vielfältig publiziert.

Nach Generierung der Mausmodelle können Veränderungen, die durch das Transgen bzw. durch den Funktionsverlust eines Gens induziert wurden, im Kontext des Gesamtorganismus analysiert werden (Balling R, 2001. Ann. Rev. Genomics Hum. Genet. 2:463-92). So sind zum Beispiel Verhaltenstests genauso wie biochemische Untersuchen etablierter Blutparameter möglich. Histologische Analysen, Immunhistochemie oder die Elektronenmikroskopie ermöglichen die Charakterisierung von Veränderungen auf zellulärer Ebene. Das spezifische Expressionsmuster eines Genes kann durch eine in situ Hybridisierung nachgewiesen werden (Peters T. et. al. 2003. Hum. Mol. Genet 12: 2109-20).

### Beispiel 1: Identifizierung der SEQ ID NO: 5/6 als diagnostisches und therapeutisches Krebs-Target

Die SEQ ID NO: 5 (Nukleinsäuresequenz) wird von einem neuen Gen auf Chromosom 11 (11q12.1) kodiert und repräsentiert die abgeleitete Proteinsequenz (SEQ ID NO 6). Ein alternatives offenes Leseraster dieses Genlokus ist SEQ ID NO: 269, die für die abgeleitete Proteinsequenz SEQ ID NO: 270 kodiert. Beide Proteinsequenzen zeigen keine Homologien zu bereits bekannten Proteinen.

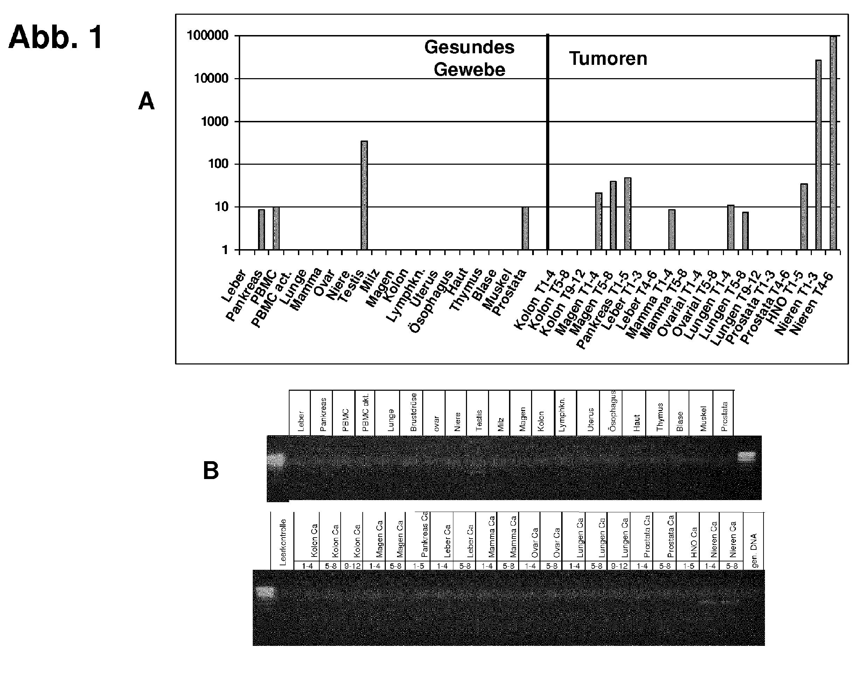

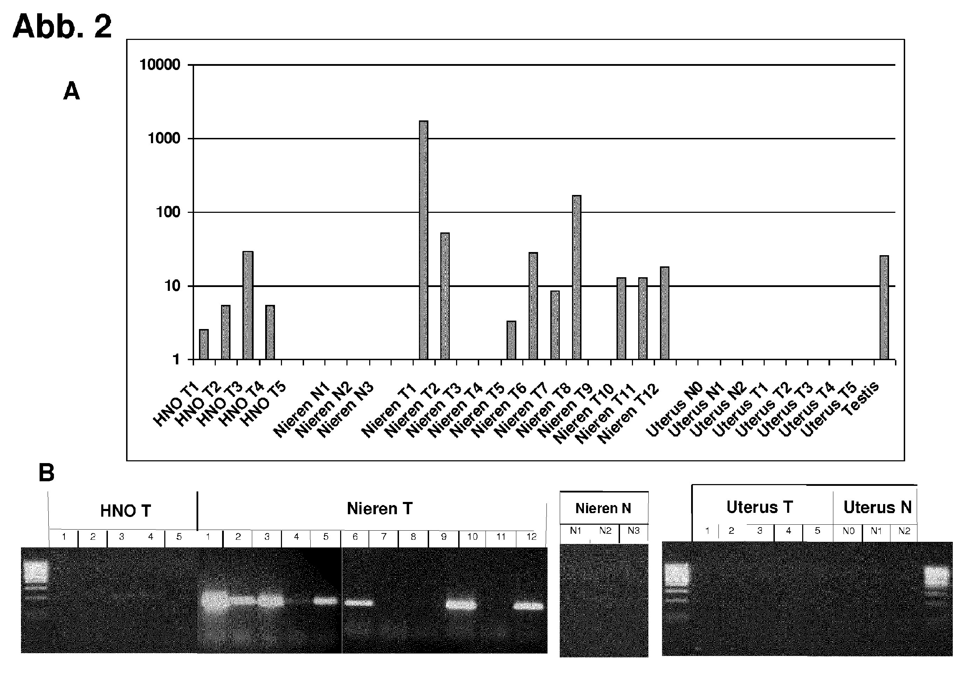

Es wurde nach der Etablierung einer genspezifischen quantitativen RT-PCR (Primerpaar SEQ ID NO: 7 und 8) die Menge der genspezifischen Transkripte in gesundem Gewebe und in Karzinomproben (jeweils Pool von Proben) untersucht. Spezifische RNA detektierten wir mit Ausnahme von Testis gar nicht oder aber nur in geringen Mengen in den von uns untersuchten gesunden Geweben (Abb. 1; A: quantitative RT-PCR; B: Gelbild). Der Lokus exprimiert demnach ein keimzellspezifisches Genprodukt mit stringenter Unterdrückung der Expression in normalen somatischen Geweben. Allerdings ist das Gen in vielen Tumorproben aktiviert, spezifische RNA war in substantiellen Mengen nachweisbar (Abb. 1 und 2). Die höchste Prävalenz und Expressionshöhe fanden wir in Nierenzelltumoren. Aber auch in Magen-, Pankreas-, HNO- und Lungentumoren waren spezifische Transkripte nachweisbar. Um die Expression von diesem Genlokus zusätzlich zu belegen, wurde zusätzlich ein Northern Blot durchgeführt. Dazu wurde eine Sonde in einer spezifischen PCR der Primer SEQ ID NO: 7 und 8 unter Einbau von Digoxygenin-dUTP (Roche Diagnostics) nach Angaben des Herstellers hergestellt. Die Sonde wurde anschließend mit 2 µg (Abb. 3a, Spur 1) bzw. 1 µg (Abb. 3a, Spur 2) Gesamt-RNA aus Testisgewebe hybridisiert und das Digoxygenin der Sonde anschließend in einer spezifischen Farbreaktion nachgewiesen. Ein ca. 3,1 kB großes, genspezifisches Fragment konnte in dem Experiment nachgewiesen (Abb. 3a) und bestätigte somit zusätzlich die Expression dieses Lokus.

Der Genlokus ist somit ein typischer Vertreter der Klasse der sog. Cancer/Testis-Antigene, die in Normalgeweben fast ausschließlich in den Keimzellen der Testis exprimiert sind. In Tumoren jedoch werden Cancer/Testis-Antigene häufig eingeschaltet, obwohl sie in den zugrunde liegenden somatischen Normalgewebszellen nicht exprimiert werden. Mehrere Mitglieder dieser funktionell und strukturell heterogenen Klasse werden aufgrund ihrer attraktiven selektiven Gewebsverteilung bereits für spezifische immuntherapeutischen Ansätze bei Krebserkrankungen in Phase I/II Studien getestet (z.B. Scanlan MJ, Gure AO, Jungbluth AA, Old LJ, Chen YT. 2002. Immunol Rev. 2002 Oct; 188: 22-32).

Zur Herstellung von Antikörpern können u.a. die Peptide SEQ ID NO: 282 und 283 genutzt werden. Insbesondere die extrazellulären Domänen der SEQ ID NO: 6 bzw. SEQ ID NO: 270 können als Zielstruktur von monoklonalen Antikörpern genutzt werden. Die Immunisierung von Tieren mit diesen Peptiden resultierte in der Verfügbarkeit von Antikörpern, mit denen wir die Daten der PCR-Untersuchung auch auf Proteinebene durch immunhistochemische Untersuchungen bestätigen konnten. Immunhistochemische Färbungen von Testis als Positivkontrolle aber auch von Nierentumoren (Abb. 3b) zeigten, dass dieses neu entdeckte Genprodukt tatsächlich zu Protein translatiert wird, dass es nicht in Normalgeweben außer Testis, aber in Tumoren nachweisbar ist, und dass Tumorzellen eines Patienten tatsächlich auf der Oberfläche angefärbt werden. Des Weiteren unterstützen diese Daten, dass dieses Genprodukt mit seiner außerordentlichen Tumorzell-Selektivität als Zielstruktur für einen therapeutisch und diagnostisch einsetzbaren Antikörper dienen kann, wie auch dass prinzipiell solche Antikörper gegen dieses Genprodukt generiert werden können.

## Patentansprüche

1. Verfahren zur Diagnose einer Krebserkrankung, die sich durch die Expression oder abnormale Expression eines Tumor-assoziierten Antigens auszeichnet, umfassend
(i) den Nachweis einer Nukleinsäure, die für das Tumor-assoziierte Antigen kodiert, in einer aus einem Patienten isolierten biologischen Probe, und/oder
(ii) den Nachweis des Tumor-assoziierten Antigens, in einer aus einem Patienten isolierten biologischen Probe,
wobei das Verfahren gemäß (ii) die Kontaktierung der biologischen Probe mit einem Antikörper und den Nachweis der Komplexbildung zwischen dem Antikörper und dem Tumor-assoziierten Antigen umfasst, wobei der Antikörper spezifisch an das Tumor-assoziierte Antigen bindet,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 5 des Sequenzprotokolls umfasst, und
(b) einer Nukleinsäure, die zu der Nukleinsäure unter (a) mindestens 90% Sequenzidentität aufweist.

2. Verfahren zur Bestimmung der Regression, des Verlaufs oder des Ausbruchs einer Krebserkrankung, die sich durch die Expression oder abnormale Expression eines Tumor-assoziierten Antigens auszeichnet, umfassend die Überwachung einer Probe aus einem Patienten, der die Erkrankung aufweist oder in Verdacht steht, an der Erkrankung zu erkranken, in Bezug auf einen oder mehrere Parameter, ausgewählt aus der Gruppe, bestehend aus:
(i) der Menge der Nukleinsäure, die für das Tumor-assoziierte Antigen kodiert,
und
(ii) der Menge des Tumor-assoziierten Antigens,
wobei das Verfahren gemäß (ii) die Kontaktierung der biologischen Probe mit einem Antikörper und den Nachweis der Komplexbildung zwischen dem Antikörper und dem Tumor-assoziierten Antigen umfasst, wobei der Antikörper spezifisch an das Tumor-assoziierte Antigen bindet,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 5 des Sequenzprotokolls umfasst, und
(b) einer Nukleinsäure, die zu der Nukleinsäure unter (a) mindestens 90% Sequenzidentität aufweist.

3. Verfahren nach Anspruch 1 oder 2, wobei der Antikörper ein monoklonaler, chimärer oder humanisierter Antikörper oder ein Fragment eines Antikörpers ist.

4. Verfahren nach einem der Ansprüche 1 bis 3, wobei die Erkrankung ein Nieren-, Brust-, Pankreaskrebs, ein Lungen-, Magen-, oder ein Mammakarzinom ist.

5. Verfahren nach einem der Ansprüche 1 bis 4, wobei das Tumor-assoziierte Antigen eine Aminosäuresequenz gemäß SEQ ID NO: 6 des Sequenzprotokolls umfasst.

6. Verwendung eines Kits zur Diagnose einer Krebserkrankung, die sich durch die Expression oder abnormale Expression eines Tumor-assoziierten Antigens auszeichnet, umfassend Mittel zum Nachweis
(i) der Nukleinsäure, die für das Tumor-assoziierte Antigen kodiert, oder
(ii) des Tumor-assoziierten Antigens,
wobei die Diagnose gemäß (ii) die Kontaktierung einer biologischen Probe mit dem Mittel und den Nachweis der Komplexbildung zwischen dem Mittel und dem Tumor-assoziierten Antigen umfasst, wobei das Mittel ein Antikörper ist, der spezifisch an das Tumor-assoziierte Antigen bindet,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 5 des Sequenzprotokolls umfasst, und
(b) einer Nukleinsäure, die zu der Nukleinsäure unter (a) mindestens 90% Sequenzidentität aufweist.

## Claims

1. A method for diagnosing a cancer disease **characterized by** expression or abnormal expression of a tumor-associated antigen, which method comprises
(i) detecting a nucleic acid which codes for the tumor-associated antigen in a biological sample isolated from a patient, and/or
(ii) detecting the tumor-associated antigen in a biological sample isolated from a patient,
wherein the method according to (ii) comprises contacting the biological sample with an antibody and detecting the formation of a complex between the antibody and the tumor-associated antigen, wherein the antibody binds specifically to said tumor-associated antigen,
said tumor-associated antigen having a sequence encoded by a nucleic acid which is selected from the group consisting of:
(a) a nucleic acid which comprises a nucleic acid sequence according to SEQ ID NO: 5 of the sequence listing, and
(b) a nucleic acid which has at least 90% sequence identity to the nucleic acid of (a).

2. A method for determining regression, course or onset of a cancer disease **characterized by** expression or abnormal expression of a tumor-associated antigen, which method comprises monitoring a sample from a patient who has said disease or is suspected of falling ill with said disease, with respect to one or more parameters selected from the group consisting of:
(i) the amount of the nucleic acid which codes for the tumor-associated antigen, and
(ii) the amount of the tumor-associated antigen,
wherein the method according to (ii) comprises contacting the biological sample with an antibody and detecting the formation of a complex between the antibody and the tumor-associated antigen, wherein the antibody binds specifically to said tumor-associated antigen,
said tumor-associated antigen having a sequence encoded by a nucleic acid which is selected from the group consisting of:
(a) a nucleic acid which comprises a nucleic acid sequence according to SEQ ID NO: 5 of the sequence listing, and
(b) a nucleic acid which has at least 90% sequence identity to the nucleic acid of (a).

3. The method of claim 1 or 2, wherein the antibody is a monoclonal, chimeric or humanized antibody, or a fragment of an antibody.

4. The method of any one of claims 1 to 3, wherein the disease is a kidney, breast or pancreatic cancer, or a lung, gastric or mammary carcinoma.

5. The method of any one of claims 1 to 4, wherein the tumor-associated antigen comprises an amino acid sequence according to SEQ ID NO: 6 of the sequence listing.

6. Use of a kit for diagnosing a cancer disease **characterized by** expression or abnormal expression of a tumor-associated antigen, which kit comprises agents for detecting
(i) the nucleic acid which codes for the tumor-associated antigen, or
(ii) the tumor-associated antigen,
wherein the diagnosis according to (ii) comprises contacting a biological sample with the agent and detecting the formation of a complex between the agent and the tumor-associated antigen, wherein the agent is an antibody which binds specifically to said tumor-associated antigen,
said tumor-associated antigen having a sequence encoded by a nucleic acid which is selected from the group consisting of:
(a) a nucleic acid which comprises a nucleic acid sequence according to SEQ ID NO: 5 of the sequence listing, and
(b) a nucleic acid which has at least 90% sequence identity to the nucleic acid of (a).

## Revendications

1. Procédé pour le diagnostic d'une affection cancéreuse **caractérisée par** l'expression ou l'expression anormale d'un antigène associé à une tumeur, lequel procédé comprend
(i) la détection d'un acide nucléique qui code pour l'antigène associé à une tumeur dans un échantillon biologique isolé à partir d'un patient, et/ ou
(ii) la détection de l'antigène associé à une tumeur, dans un échantillon biologique isolé à partir d'un patient,
tandis que le procédé selon (ii) comprend la mise en contact de l'échantillon biologique avec un anticorps et la détection de la formation d'un complexe entre l'anticorps et l'antigène associé à une tumeur, tandis que l'anticorps se lie spécifiquement audit antigène associé à une tumeur,
ledit antigène associé à une tumeur ayant une séquence codée par un acide nucléique qui est choisi dans le groupe consistant en :
(a) un acide nucléique qui comprend une séquence d'acide nucléique selon SEQ ID NO : 5 du listage de séquences, et
(b) un acide nucléique qui a au moins 90% d'identité de séquences vis-à-vis de l'acide nucléique de (a).

2. Procédé pour la détermination de la régression, du déroulement ou de l'apparition d'une affection cancéreuse **caractérisée par** l'expression ou l'expression anormale d'un antigène associé à une tumeur, lequel procédé comprend la surveillance d'un échantillon provenant d'un patient qui a ladite affection ou est suspecté d'être atteint de cette affection, vis-à-vis d'un ou plusieurs paramètres choisis dans le groupe consistant en:
(i) la quantité de l'acide nucléique qui code pour l'antigène associé à une tumeur, et
(ii) la quantité de l'antigène associé à une tumeur,
dans lequel le procédé selon (ii) comprend la mise en contact de l'échantillon biologique avec un anticorps et la détection de la formation d'un complexe entre l'anticorps et l'antigène associé à une tumeur, tandis que l'anticorps se lie spécifiquement audit antigène associé à une tumeur,
ledit antigène associé à une tumeur ayant une séquence codée par un acide nucléique qui est choisi dans le groupe consistant en:
(a) un acide nucléique qui comprend une séquence d'acide nucléique selon SEQ ID NO: 5 du listage de séquences, et
(b) un acide nucléique qui a au moins 90% d'identité de séquences vis-à-vis de l'acide nucléique de (a).

3. Procédé selon la revendication 1 ou 2, dans lequel l'anticorps est un anticorps monoclonal, chimérique ou humanisé, ou un fragment d'un anticorps.

4. Procédé selon l'une quelconque des revendications 1 à 3, dans lequel l'affection est un cancer du rein, du sein ou du pancréas, ou un carcinome pulmonaire, gastrique ou mammaire.

5. Procédé selon l'une quelconque des revendications 1 à 4, dans lequel l'antigène associé à une tumeur comprend une séquence d'acides aminés selon SEQ ID NO: 6 du listage de séquences.

6. Utilisation d'un kit pour le diagnostic d'une affection cancéreuse **caractérisée par** l'expression ou l'expression anormale d'un antigène associé à une tumeur, lequel kit comprend des agents pour la détection
(i) de l'acide nucléique qui code pour l'antigène associé à une tumeur, ou
(ii) de l'antigène associé à une tumeur,
dans laquelle diagnostic selon (ii) comprend la mise en contact d'un échantillon biologique avec l'agent et la détection de la formation d'un complexe entre l'agent et l'antigène associé à une tumeur, tandis que l'agent est un anticorps qui se lie spécifiquement audit antigène associé à une tumeur,
ledit antigène associé à une tumeur ayant une séquence codée par un acide nucléique qui est choisi dans le groupe consistant en:
(a) un acide nucléique qui comprend une séquence d'acide nucléique selon SEQ ID NO: 5 du listage de séquences, et
(b) un acide nucléique qui a au moins 90% d'identité de séquences vis-à-vis de l'acide nucléique de (a).
